# EUROPEAN PATENT SPECIFICATION

(11) **EP 2 945 947 B1**
(45) Date of publication and mention of the grant of the patent: **04.12.2019**
(21) Application number: 14741091.4
(22) Date of filing: 17.01.2014
(51) Int. Cl.: C07D 473/08, C07F 9/09

(54) **INHIBITING THE TRANSIENT RECEPTOR POTENTIAL A1 ION CHANNEL**
IONENKANALHEMMUNG DES ÜBERGANGSREZEPTORPOTENTIALS A1
INHIBITION DU CANAL IONIQUE À POTENTIEL DE RÉCEPTEUR TRANSITOIRE A1

(30) Priority: 18.01.2013 US 201361754103 P; 18.01.2013 US 201361754132 P; 13.03.2013 US 201361780836 P; 26.04.2013 US 201361816290 P
(43) Date of publication of application: 25.11.2015
(73) Proprietor: Merck Sharp & Dohme Corp., Rahway, NJ 07065 (US)
(72) Inventor: LI, Qingyi, Somerville, MA 02143 (US); CHENG, Lily, Chestnut Hill, MA 02467 (US); LIU, Christopher, Somerville, MA 02144 (US); WRONA, Iwona, Sharon, MA 02067 (US); LIPPA, Blaise, S., Acton, MA 01720 (US); METCALF, Chester, A. III, Needham, MA 02492 (US); JACKSON, Andrew, J., Waltham, MA 02451 (US)
(74) Representative: Jaap, David Robert
(86) International application number: PCT/US2014/012049
(87) International publication number: WO 2014/113671

(56) References cited:
- WO-A1-2009/140517
- WO-A1-2010/036821
- WO-A1-2013/023102
- US-A1- 2010 249 154
- US-A1- 2011 151 018
- US-B2- 7 582 658
- US-B2- 8 163 761

## Description

### TECHNICAL FIELD

Provided herein are pharmaceutical compositions and compounds for use in the treatment of pain and respiratory conditions, as well as inhibiting the Transient Receptor Potential A1 (TRPA1) ion channel.

### BACKGROUND

Transient Receptor Potential A1 (herein, "TRPA1") is a non-selective cation channel related to pain sensation in humans. TRPA1 is found in sensory neurons and functions as a signal transduction receptor linking inflammation to pain. Activation of TRPA1 is believed to cause pain by inducing firing of nociceptive neurons and driving central sensitization in the spinal cord. TRPA1 stimulation can also increase firing of sensory neurons, leading to the release of pro-inflammatory neuropeptides such as NK-A, substance P and CGRP (which induce vasodilation and help recruit immune cells). A variety of endogenous reactive compounds produced during inflammation activate TRPA1 (including 4-hydroxynonenal released during liposome peroxidation; cyclopentane prostaglandins synthesized by COX enzymes; hydrogen peroxide produced by oxidative stress). Activation of TRPA1 also sensitizes TRPA1 to cold. Furthermore, a gain-of-function mutation in TRPA1 causes familial episodic pain syndrome; patients suffering from this condition have episodic pain that may be triggered by cold. Thus, TRPA1 is considered to play a role in pain related to nerve damage, cold allodynia, and inflammatory pain.

Compounds that inhibit the TRPA1 ion channel can be useful, for example, in treating conditions ameliorated, eliminated or prevented by inhibition of the TRPA1 ion channel. For example, pharmaceutical compositions that inhibit TRPA1 can be used to treat pain. Inhibition of TRPA1 (e.g., by genetic ablation and chemical antagonism) has been shown to result in reduced pain behavior in mice and rats. Knockout mice lacking functional TRPA1 have diminished nociceptive responses to TRPA1 activators (including AITC, formalin, acrolein, 4-hydroxynonenal) and, in addition, have greatly reduced thermal and mechanical hypersensitivity in response to the inflammatory mediator bradykinin (e.g., Kwan, K. Y. et al. Neuron 2006, 50, 277-289; Bautista, D. M. et al. Cell 2006, 124, 1269-1282). In animal pain models, down regulation of TRPA1 expression by gene specific antisense sequences prevented and reversed cold hyperalgesia induced by inflammation and nerve injury (See, e.g., Obata, K. et al., Journal of Clinical Investigation 2005, 115, 2393-2401; Jordt, S. E. et al., Nature 2004, 427, 260-265; Katsura, H. et al., Exploratory Neurology 2006, 200, 112-123). TRPA1 inhibitor compounds are effective in a variety of rodent pain models. TRPA1 inhibitors have been shown to reduce mechanical hypersensitivity and cold allodynia following inflammation induced by Complete Freund's Adjuvant (without altering normal cold sensation in naive animals) and also to improve function in the rat mono-iodoacetate osteoarthritis model (Materazzi, S et al., European Journal of Physiology 2012, 463(4):561-9; Wei H et al., Anesthesiology 2012, 117(1):137-48; Koivisto, A et al., Pharmacol Res. 2012, 65(1):149-58). TRPA1 inhibitor compounds have demonstrated reduced pain behavior in rodents injected with AITC (mustard oil), formalin, cinnamaldehyde, acrolein, and other TRPA1 activators.

A compound for inhibiting the TRPA1 ion channel is disclosed in WO2010/075353A1:

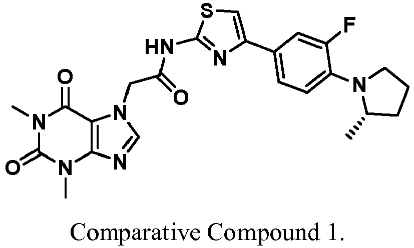

However, while Comparative Compound 1 is disclosed as an inhibitor of the TRPA1 ion channel, the administration of Comparative Compound 1 was later found to elevate the serum biomarkers of hepatotoxicity in certain animal studies (e.g., as disclosed in Example 5 herein).

Another compound for inhibiting the TRPA1 ion channel is disclosed in WO2013/023102A1:

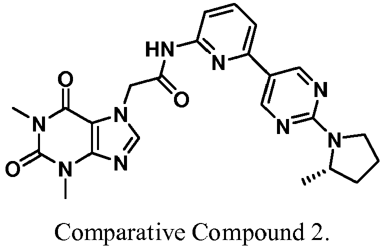

While Comparative Compound 2 is an inhibitor of the TRPA1 ion channel that did not elevate the serum biomarkers of hepatotoxicity in the animal studies (as disclosed in Example 5 herein), Comparative Compound 2 has undesirably low TrpA1 potency, and low aqueous solubility for formulation in intravenous administration (see data in Example 2 herein).

Accordingly, there remains an unmet medical need for novel compounds with greater potency and/or aqueous solubility than Comparative Compound 2 (e.g., for use in intravenous or oral pharmaceutical compositions) that are therapeutically effective in the treatment of pain, respiratory conditions, and/or the inhibition of the TRPA1 ion channel, without producing serum biomarkers of hepatotoxicity observed when administering the Comparative Compound 1 (e.g., in the method of Example 5 disclosed herein). Such novel compounds preferably inhibit the TRPA1 ion channel *in vivo,* without producing serum biomarkers of hepatotoxicity.

### SUMMARY OF THE INVENTION

Compounds of Formula (Ia) are useful for the treatment of pain and/or to inhibit the TRPA1 ion channel: 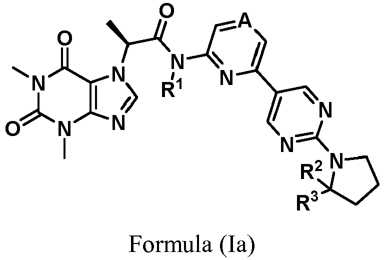 wherein
R¹ is hydrogen;
R² is hydrogen or CH₃;
R³ is CH₃ or CF₃; and
A is N or CH.

In another embodiment of Formula (Ia), R² is hydrogen and R³ is CF₃. In yet another embodiment of Formula (Ia), R² is CH₃ and R³ is CH₃. In particular embodiments of Formula (Ia), A is N.

In preferred embodiments of Formula (Ia), the compound is selected from a group consisting of:
(*S*)-(2-(1,3-dimethyl-2,6-dioxo-1,2,3,6-tetrahydro-7*H*-purin-7-yl)-*N*-(6-(2-(2,2-dimethylpyrrolidin-1-yl)pyrimidin-5-yl)pyrazin-2-yl)propanamide;
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyridin-2-yl)propanamide;
(*S*)-2-(1,3-dimethyl-2,6-dioxo-1,2,3,6-tetrahydro-7*H*-purin-7-yl)-*N*-(6-(2-((S)-2-methylpyrrolidin-1 -yl)pyrimidin-5 -yl)pyrazin-2-yl)propanamide; and
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyrazin-2-yl)propanamide;
or a pharmaceutically acceptable salt thereof.

The present invention is based in part on the unexpected discovery that compounds of Formula (Ia) can inhibit the TRPA1 ion channel (according to the method of Example 2 disclosed herein) without elevating serum biomarkers of hepatotoxicity (according to the method of Example 5 disclosed herein). Described also are compounds of Formula (Ia) where R¹ is CH₂-R^{1a}, and R^{1a} is a phosphate moiety which have improved aqueous solubility compared to Comparative Compound 2 (including compounds with aqueous solubility suitable for pharmaceutical compositions formulated for intravenous administration). Further, compounds of Formula (Ia) have improved potency against TrpA1. Without being bound by any particular theory, it is believed that the methyl group between the xanthine and the amide groups in compounds of Formula (Ia) surprisingly provides a significant boost to *in vitro* and *in vivo* TrpA1 potency. Upon administration, compounds of Formula (Ia) where R¹ is CH₂-R^{1a}, and R^{1a} is a phosphate moiety can be administered to therapeutically reduce symptoms of pain. Administration of compounds of Formula (Ia) where R¹ is CH₂-R^{1a}, and R^{1a} is a phosphate moiety can result in the *in vivo* conversion into compounds of Formula (Ia) where R¹ is H in amounts effective to therapeutically treat pain (e.g., by inhibiting the TRPA1 ion channel). Compounds of Formula (Ia) are also useful in amounts effective to therapeutically treat respiratory conditions such as obstructive diseases, e.g., chronic obstructive pulmonary disease (COPD), asthma (e.g., cold induced asthma, exercise-induced asthma, allergy-induced asthma, and occupational asthma), and cough, preferably a condition responsive to a TRPA1 inhibitor.

Thus, provided herein is a compound of Formula (Ia), or a pharmaceutical composition comprising a compound of Formula (Ia) for use in treating or ameliorating a pulmonary disease in an animal or human.

In some embodiments, the pulmonary disease is asthma. In particular embodiments, the asthma is allergic asthma. In other embodiments, the disease is chronic obstructive pulmonary disease (COPD).

In addition, compounds of Formula (Ia) are useful in the manufacture of a pharmaceutical composition formulated for intravenous administration to treat pain (e.g., a prodrug compound of Formula (Ia) where R¹ is CH₂-R^{1a}, and R^{1a} is a phosphate moiety that can be converted within the blood of a mammal to a compound of Formula (Ia) where R¹ is H after administration).

Also provided herein is a compound of Formula (Ia), or a pharmaceutical composition comprising a compound of Formula (Ia) for use in treating pain or providing analgesia, In some embodiments, the compound is intravenously or orally administered to the subject in need thereof.

In one embodiment, the pain is post-surgical pain or chronic pain. In yet another embodiment, the pain is inflammatory pain.

### BRIEF DESCRIPTION OF THE DRAWINGS

**Figure 1** is a line graph demonstrating increased Paw Withdrawal Latency scores observed after intravenous (i.v.) administration of pharmaceutical compositions with increasing concentrations of a compound of Formula (Ia) in the Complete Freund's Adjuvant rodent model of Example 4.
**Figure 2** is a line graph demonstrating Paw Withdrawal Latency scores for Comparator Compound 2.
**Figure 3A** is a line graph depicting the time course of antigen-induced changes in lung resistance (RL) in sheep treated with Vehicle or 1 mg/kg Compound 5-T. BL = baseline. Values are mean ± se for 3 sheep.
**Figure 3B** is a bar graph demonstrating the effect of Vehicle or 1 mg/kg Compound 5-T on airway responsiveness. A decrease in the PC 400 indicates the development of airway hyperresponsiveness. Values are mean ± se for 3 sheep. BL = baseline; 24hPA = 24h post antigen. Statistical analysis is provided in Table 4.
**Figure 4A** is a line graph demonstrating the time course of antigen-induced changes in lung resistance (RL) in sheep treated with 3 or 10 mg/kg Compound 5-T. BL = baseline. Values are mean ± se for 3 sheep.
**Figure 4B** is a bar graph depicting: the effect of 3 and 10 mg/kg of Compound 5-T on airway responsiveness. A decrease in the PC 400 indicates the development of airway hyperresponsiveness. Values are mean ± se for 3 sheep. BL = baseline. Statistical analysis is provided in Table 4.
**Figure 5** is a line graph demonstrating the percent inhibition of antigen-induced responses with different doses of Compound 5-T. Values were calculated from results in Table 4.

### DETAILED DESCRIPTION

When a stereoisomer is depicted by name or structure, it is to be understood that the stereoisomeric purity of the named or depicted stereoisomer is at least 60%, 70%, 80%, 90%, 99% or 99.9% pure by weight. Stereoisomeric purity is determined by dividing the weight of the named or depicted stereoisomer in the mixture by the total weight of all stereoisomers in the mixture.

When a compound is designated by a name or structure that indicates a single enantiomer, unless indicated otherwise, the compound is at least 60%, 70%, 80%, 90%, 99% or 99.9% optically pure (also referred to as "enantiomerically pure"). Optical purity is the weight in the mixture of the named or depicted enantiomer divided by the total weight in the mixture of both enantiomers.

In addition, compounds of Formula (Ia) can include one or more isotopes of the atoms present in Formula (Ia). For example, compounds of Formula (Ia) can include: those in which H (or hydrogen) is replaced with any isotopic form of hydrogen including ¹H, ²H or D (Deuterium), and ³H (Tritium); those in which C is replaced with any isotopic form of carbon including ¹²C, ¹³C, and ¹⁴C; those in which N is replaced with any isotopic form of nitrogen including ¹³N, ¹⁴N and ¹⁵N; those in which F is replaced with any isotopic form of fluorine including ¹⁹F and ¹⁸F; and the like. In a preferred embodiment, compounds represented by Formula (Ia) comprise isomers of the atoms therein in their naturally occurring abundance. In particular embodiments of the compounds of Formula (Ia) when, for example, hydrogen is enriched in the deuterium isotope, the symbol "D" is used to represent the enrichment in deuterium.

In one embodiment of Formula (Ia), R² is hydrogen and R³ is CF₃. In yet another embodiment of Formula (Ia), R² is CH₃ and R³ is CH₃.

In particular embodiments of Formula (Ia), A is N.

Compounds of Formula (Ia) include molecules having an aqueous solubility suitable for intravenous administration leading to or resulting in the treatment of pain symptoms and/or inhibition of the TRPA1 ion channel with a therapeutically effective potency. The potency of compounds of Formula (Ia) in inhibiting the TRPA1 ion channel was measured using the method of Example 1. Tables 1a - f disclose the TRPA1 inhibition *in vitro* potency (measured by the method of Example 1). Preferred compounds of Formula (Ia) include compounds that inhibit the TRPA1 ion channel with a IC₅₀ value obtained by the method of Example 1 of less than about 100 nM (preferably, less than about 76 nM, more preferably less than about 25 nM) and most preferably less than to the comparable measurement of Comparator Compound 2. Tables 1a - f show data obtained from the *in vitro* assay described in Example 1, where an "A" value represents an IC₅₀ value of less than 25 nanomolar (nM); a "B" value represents an IC₅₀ value between 25 nM and 75 nM; a "C" value represent an IC₅₀ value between 76 nM and 100 nM; and a "D" value represents an IC₅₀ value exceeding 100 nM.

Compounds of Formula (Ia) where R¹ is H can inhibit the TRPA1 ion channel. Compounds of Formula (Ia) are provided in compositions with more of a compound of Formula (Ia) than the corresponding stereoisomer(s). For example, a pharmaceutical composition can contain a therapeutically effective amount of a compound of Formula (Ia) in an excess over the corresponding stereoisomers (e.g., a compound of Formula (Ia) represents more than at least about 50% and preferably 78%, 85%, 90%, 95% or more of the total amount of compounds of Formula (Ia) in a composition). Compounds of Formula (Ia) (preferably compounds of Formula (Ia)) where R¹ is CH₂-R^{1a}, and R^{1a} is a phosphate moiety can be administered as part of an intravenous pharmaceutical composition to treat pain in therapeutically effective manner. Upon intravenous administration, compounds of Formula (Ia) where R¹ is CH₂-R^{1a}, and R^{1a} is a phosphate moiety can be converted *in vivo* into compounds of Formula (Ia) that therapeutically inhibit the TRPA1 ion channel (e.g., where R¹ is H).

Compound 5-T inhibits the TRPA1 ion channel. A pharmaceutical composition can contain a therapeutically effective amount of a compound 5-T in an excess over the corresponding stereoisomers of, for example compound 5-V, (e.g., a compound 5-T represents more than at least about 50% and preferably 78%, 85%, 90%, 95% or more of the total amount of the compounds in a composition). Compound 5-T can be administered as part of an intravenous or oral pharmaceutical composition to treat pain in a therapeutically effective manner.

Compound 1-B inhibits the TRPA1 ion channel. A pharmaceutical composition can contain a therapeutically effective amount of a compound 1-B in an excess over the corresponding stereoisomers of, for example compound 1-D, (e.g., a compound 1-B represents more than at least about 50% and preferably 78%, 85%, 90%, 95% or more of the total amount of the compounds in a composition). Compound 1-B can be administered as part of an intravenous or oral pharmaceutical composition to treat pain in therapeutically effective manner.

Compound 3-K inhibits the TRPA1 ion channel. A pharmaceutical composition can contain a therapeutically effective amount of a compound 3-K in an excess over the corresponding stereoisomers of, for example compound 3-L, (e.g., a compound 3-K represents more than at least about 50% and preferably 78%, 85%, 90%, 95% or more of the total amount of the compounds in a composition). Compound 3-K can be administered as part of an intravenous or oral pharmaceutical composition to treat pain in therapeutically effective manner.

Tables 1a - f also disclose the aqueous solubility measured at various pH values (using the method of Example 2) for various compounds of Formula (Ia) and certain comparator compounds. Unless otherwise indicated herein, the aqueous solubility of certain compounds of Formula (Ia) was measured using the method of Example 2. Preferred compounds of Formula (Ia) include compounds having an aqueous solubility of greater than about 0.001 mg/mL (preferably, greater than about 1 mg/mL) at a therapeutically relevant pH (e.g., about pH 2-9, and preferably about pH 7 for intravenous administration), as measured by the method of Example 2. In addition, the data in Tables 1a - f show compounds of Formula (Ia) where R¹ is CH₂-R^{1a}, and R^{1a} is a phosphate moiety have improved aqueous solubility compared to Comparator Compound 2 as measured by the method of Example 2.

Referring to Tables 1a - f, compositions comprising compounds of Formula (Ia) where R¹ is H have improved potency in inhibiting the TRPA1 ion channel using the method of Example 1, compared to Comparative Compounds 2 - 6. Specific compounds of Formula (Ia) include molecules where A is CH, R¹ is hydrogen or CH₂-R^{1a} (R^{1a} being a phosphate moiety), R³ is CF₃, and R² is H, including compounds shown in Table 1b. Additional compounds of Formula (Ia) include molecules where A is N, R¹ is hydrogen or CH₂-R^{1a} (R^{1a} being a phosphate moiety), R³ is CF₃, and R² is H, including compounds shown in Table 1c. Compounds of Formula (Ia) also include molecules where A is N, R¹ is hydrogen or CH₂-R^{1a} (R^{1a} being a phosphate moiety), R² is CH₃, and R³ is CH₃, including compounds shown in Table 1d. Compounds of Formula (Ia) where A is N, R¹ is hydrogen or CH₂-R^{1a} (R^{1a} being a phosphate moiety), R³ is CH₃, and R² is H include compounds shown in Table 1e. Additional compounds of Formula (Ia) are molecules where A is CH, R¹ is hydrogen or CH₂-R^{1a} (R^{1a} being a phosphate moiety), R³ is CH₃, and R² is H, including compounds shown in Table 1f.

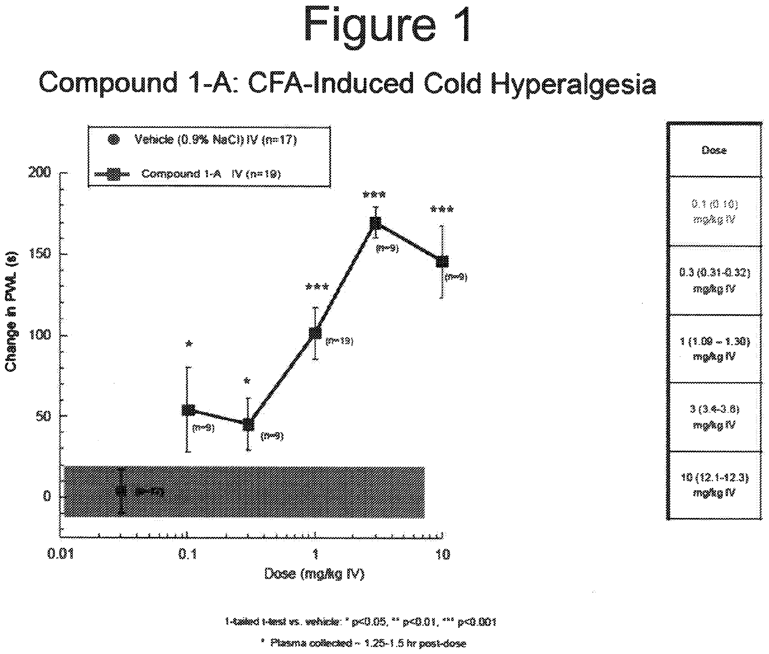

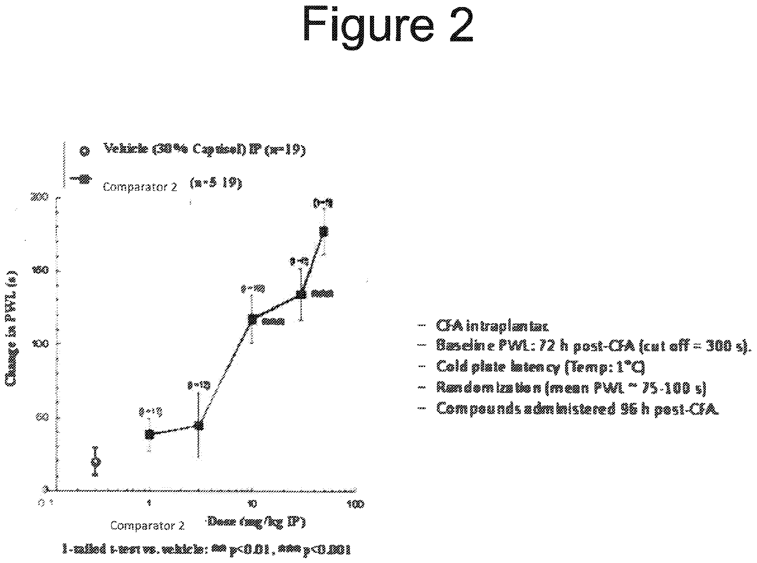

Compounds of Formula (Ia) were effective in treating pain, as evaluated by the methods of Example 3 (rodent formalin pain model) and Example 4 (rodent cold plate pain model). Table 2 discloses data obtained by testing compounds of Formula (Ia) and a control (vehicle) administered without a compound of Formula (Ia) according to the method of Example 3. The rodent formalin-induced pain model data in Table 2 shows that compounds of Formula (Ia) reduced the duration of pain behavior observed for the control in the absence of a compound of Formula (Ia) (i.e., the duration of pain behavior was less than 88 minutes observed for the control). Figure 1 is a graph showing the results obtained by testing a compound of Formula (Ia) (i.e., Compound 1-A, structure in Table 2) and a vehicle (without a compound of Formula (Ia)) in a rodent cold plate pain model described in Example 4. The data shown in Figure 1 shows increased rodent Paw Withdrawal Latency (PWL) observed after i.v. administration of pharmaceutical compositions of Compound 1-A in the Complete Freud's Adjuvant rodent model. This data was obtained by measuring the change in PWL score as a function of the concentration of Compound 1-A, as well as a control vehicle composition, as described in Example 4. The data in Figure 1 shows that compounds of Formula (Ia) reduce the symptoms of pain as measured according to the method of Example 3, and that a compound of Formula (Ia) (Compound 1-A) has an analgesic effect on cold allodynia as measured according to the method of Example 4. Figure 2 shows Cold Plate Model data obtained for Comparator Compound 2.

Compounds of Formula (Ia) shown in Table 3 and certain comparator compounds were evaluated in a rodent acute tolerability test described in Example 5, showing alteration in liver function tests (LFTs) were observed by measurements of alanine aminotranferease (ALT) and aspartate aminotranferease (AST). Unexpectedly, in contrast to the Comparator Compound in Table 3, intravenous administration of the compounds of Formula (Ia) as indicated in Table 3 did not result in elevation of either ALT or AST levels in the blood. Results in Table 3 are provided as a percentage of the highest normal expected concentrations of ALT and AST in the tested animals (i.e., 40 mg/dL ALT and 90 mg/dL AST). The data in Table 3 shows that intravenous bolus dose of the Comparator Compounds 1 resulted in significant subsequent increases in blood levels of ALT and AST to levels more than double (i.e., about 170-1,311%) of the high end of the normal ranges of these compounds prior to administration of the compounds. Preferred compounds of Formula (I) result in an ALT and/or AST level measured by the method of Example 5 of less than about 100 mg/dL one day after administration.

Examples of compounds of Formula (Ia) when R¹ is CH₂-R^{1a} (R^{1a} being a phosphate moiety) that did not result in abnormally high levels of LFT compounds ALT or AST as measured according to Example 5 include a compound selected from the group consisting of:
(*S*)-(2-(1,3-dimethyl-2,6-dioxo-1,2,3,6-tetrahydro-7*H*-purin-7-yl)-*N*-(6-(2-(2,2-dimethylpyrrolidin-1-yl)pyrimidin-5-yl)pyrazin-2-yl)propanamido)methyl dihydrogen phosphate;
((*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyridin-2-yl)propanamido)methyl dihydrogen phosphate;
((*S*)-2-(1,3-dimethyl-2,6-dioxo-1,2,3,6-tetrahydro-7*H*-purin-7-yl)-*N*-(6-(2-((*S*)-2-methylpyrrolidin-1-yl)pyrimidin-5-yl)pyrazin-2-yl)propanamido)methyl dihydrogen phosphate;
((*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamido)methyl dihydrogen phosphate; and pharmaceutically acceptable salts thereof.

These compounds can be converted within a mammal (e.g., within the blood) into a compound of Formula (Ia) where R¹ is hydrogen to form a structurally corresponding compound selected from the group consisting of:
(*S*)-2-(1,3-dimethyl-2,6-dioxo-1,2,3,6-tetrahydro-7*H*-purin-7-yl)-*N*-(6-(2-(2,2-dimethylpyrrolidin-1-yl)pyrimidin-5-yl)pyrazin-2-yl)propanamide;
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyridin-2-yl)propanamide;
(S)-2-(1,3-dimethyl-2,6-dioxo-1,2,3,6-tetrahydro-7*H*-purin-7-yl)-N-(6-(2-((S)-2-methylpyrrolidin-1-yl)pyrimidin-5-yl)pyrazin-2-yl)propanamide,
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyrazin-2-yl)propanamide,
and pharmaceutically acceptable salts thereof.

Thus, provided herein are compounds of Formula (Ia) where R¹ is hydrogen, wherein compound selected from the group consisting of:
(*S*)-2-(1,3-dimethyl-2,6-dioxo-1,2,3,6-tetrahydro-7*H*-purin-7-yl)-*N*-(6-(2-(2,2-dimethylpyrrolidin-1-yl)pyrimidin-5-yl)pyrazin-2-yl)propanamide;
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyridin-2-yl)propanamide;
(*S*)-2-(1,3-dimethyl-2,6-dioxo-1,2,3,6-tetrahydro-7*H*-purin-7-yl)-*N*-(6-(2-((S)-2-methylpyrrolidin-1-yl)pyrimidin-5-yl)pyrazin-2-yl)propanamide,
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyrazin-2-yl)propanamide,
and pharmaceutically acceptable salts thereof.
wherein the compound is substantially isolated.

In certain embodiments, the compound is selected from a group consisting of:
(*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyridin-2-yl)propanamide;
(*S*)-2-(1,3-dimethyl-2,6-dioxo-1,2,3,6-tetrahydro-7*H*-purin-7-yl)-*N*-(6-(2-((*S*)-2-methylpyrrolidin-1 -yl)pyrimidin-5 -yl)pyrazin-2-yl)propanamide; and
(*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamide;
or a pharmaceutically acceptable salt thereof
wherein the compound is substantially isolated.

In a particular embodiment, the compound is (*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamide, or a pharmaceutically acceptable salt thereof
wherein the compound is substantially isolated.

Compounds of Formula (Ia) may be synthesized in a variety of ways. The processes shown in Scheme 1 (Example 8) and Schemes 2, 3, and 4 (Example 9) are exemplary methods for synthesizing compounds of Formula (I). The reactions can be performed in solvents appropriate to the reagents and materials employed and are suitable for the transformations being effected. Disclosed reaction conditions, including choice of solvent, reaction atmosphere, reaction temperature, duration of the experiment and workup procedures, are chosen to be the conditions suitable for each reaction, as recognized by one skilled in a relevant technology. As understood by one skilled in the art of organic synthesis, the functionality present on various portions of the molecule can be selected or modified from recited examples herein to be compatible with various reagents and reactions to obtain other desired products. Such restrictions to the substituents, which are compatible with the reaction conditions, will be readily apparent to one skilled in the art and alternate methods must then be used.

Referring to Scheme 1, intermediates useful for the synthesis of compounds of Formula (Ia) can be formed by: (1) reacting an alpha mono- or di-alkyl substituted pyrrolidine (i) with 5-bromo-2-chloropyrimidine to form compound (ii), (2) reacting compound (ii) with bis(pinacolato)diboron in the presence of a suitable catalyst (e.g., palladium) to form compound (iii), and (3) coupling compound (iii) with compound (iv) in the presence of a suitable (e.g., palladium) catalyst to form intermediate (v) (where X can be N or CH).

Scheme 2 provides a reaction scheme for synthesizing a compound of Formula (Ia) when R¹ is hydrogen, using compound (v) as starting material (X being N or CH). As further described in the Examples, compound (v) can be coupled with (*S*)-2-(1,3-dimethyl-2,6-dioxo-1,2,3,6-tetrahydro-7*H*-purin-7-yl)propanoic acid under appropriate conditions and in the presence of reagents such as DIC or triphenylphosphine and DIPEA at room temperature.

Scheme 3 is a synthetic route for synthesizing compounds of Formula (Ia) when R¹ is CH₂-R^{1a}, where R^{1a} is a phosphate moiety. The route can include: (1) thioesterification of the compound (v) with NaS-R⁴ (wherein R⁴ is C₁₋₄-alkyl) to form compound (vi), (2) coupling compound (vi) with 2-(1,3-dimethyl-2,6-dioxo-1,2,3,6-tetrahydro-7*H*-purin-7-yl)acetic acid to form compound (vii), (3) methylating compound (vii) with a suitable methylating agent such as iodomethane to form compound (viii), and (4) formation of the pro-drug by one or more reactions to form the compound of Formula (I). Compound (viii) can be chirally purified before or after conversion to Formula (I) when R¹ is CH₂-R^{1a}, where R^{1a} is a phosphate moiety.

The compounds of Formula (Ia) are also useful in the manufacture of a pharmaceutical composition formulated for intravenous and oral administration to treat pain and pulmonary diseases (e.g., a prodrug compound of Formula (Ia) where R¹ is CH₂-R^{1a}, and R^{1a} is a phosphate moiety that can be converted to another compound of Formula (Ia) where R¹ is H *in vivo* after administration). A pharmaceutical composition formulated for oral and intravenous administration for the treatment of pain and pulmonary diseases can include a therapeutically effective amount of compound of Formula (Ia) shown in Tables 1a - f where R¹ is H or CH₂-R^{1a} (R^{1a} being a phosphate moiety). The amount of the compound of Formula (Ia) and indicated method of administration of the pharmaceutical composition can be selected to provide to a subject in need thereof a therapeutically effective amount of a compound of Formula (Ia) where R¹ is hydrogen within the subject after administration of the pharmaceutical composition (e.g., by *in vivo* conversion of the compound of Formula (Ia) where R¹ is CH₂-R^{1a} (R^{1a} being a phosphate moiety) to a therapeutically effective amount of compound of Formula (Ia) where R¹ is hydrogen within the body of the subject). For example, a pharmaceutical composition comprising Compound 1-A, 5-S, 2-E or 3-J can have an aqueous solubility suitable for intravenous administration. Upon intravenous administration to a subject in need thereof, the Compound 1-A, 5-S, 2-E or 3-J can be converted to a corresponding stereoisomer 1-B, 5-T, 2-F or 3-K effective for treatment of pain (e.g., by inhibiting the TRPA1 ion channel with a therapeutically effective potency).

Pharmaceutical compositions containing compounds of Formula (Ia) or pharmaceutically acceptable salts thereof can be used to treat or ameliorate medical conditions responsive to the inhibition of the TRPA1 ion channel in subjects (e.g., humans and animals). For example, the pharmaceutical compositions comprising compounds of Formula (Ia), or pharmaceutically acceptable salts thereof, are useful as a perioperative analgesic, for example in the management of mild to moderate acute post-operative pain and management of moderate to severe acute pain as an adjunct to opioid analgesics. The pharmaceutical compositions comprising a therapeutically-effective dose of compounds of Formula (Ia), can be administered to a patient for treatment of pain in a clinically safe and effective manner, including one or more separate administrations of the pharmaceutical compositions comprising compounds of Formula (Ia). For example, a pharmaceutical composition comprising a therapeutically effective dose of compounds of Formula (Ia), or pharmaceutically acceptable salts thereof can be administered (e.g., intravenously or orally) to a subject in need thereof multiple times per day (e.g., BID) over a course of treatment of one or more days to treat pain and/or pulmonary disease in the subject.

Thus, provided herein is a compound of Formula (Ia) for use in treating pain or providing analgesia, in a subject. In one embodiment, the pain is post-surgical pain. In another embodiment, the pain is chronic pain. In another embodiment, the pain is inflammatory pain.

In one embodiment, the compound of Formula (Ia) is (*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyridin-2-yl)propanamide, or a pharmaceutically acceptable salt thereof. In another embodiment, the compound of Formula (Ia) is (*S*)-2-(1,3-dimethyl-2,6-dioxo-1,2,3,6-tetrahydro-7*H*-purin-7-yl)-*N*-(6-(2-((*S*)-2-methylpyrrolidin-1-yl)pyrimidin-5-yl)pyrazin-2-yl)propanamide, or a pharmaceutically acceptable salt thereof. In yet another embodiment, the compound of Formula (Ia) is (*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamide, or a pharmaceutically acceptable salt thereof.

Pharmaceutical compositions containing compounds of Formula (Ia) (e.g., Formula (Ia)) or pharmaceutically acceptable salts thereof can also be used to treat or ameliorate respiratory conditions, such as obstructive diseases, e.g., chronic obstructive pulmonary disease (COPD), asthma (e.g., cold induced asthma, exercise-induced asthma, allergy-induced asthma, and occupational asthma), and cough.

Compounds of Formula (Ia) were evaluated in an allergic asthma model (Example 6). At the doses tested, Compound 5-T showed improved inhibition of the average early airway response (EAR) compared to Comparative Compound 2. Compound 5-T provided dose dependent inhibition of the average late airway response (LAR) and the airway hyperresponsiveness (AHR). While the protection seen on the LAR and the AHR were consistent with the proposed mechanism of action of this compound class, the effect on the EAR is not. Without being bound by any particular theory, two potential schemes that may explain these results include: a) Compound 1-B interfering with mast cell mediator release (the driver of the EAR); or b) the compound's action on airway smooth muscle. In the latter case the compound could be down regulating smooth muscle responses to the mast cell mediators which drive the EAR. In either case the physiological readout (EAR) would be affected in the same way.

Thus, provided herein is a compound of Formula (Ia) for use in treating or ameliorating pulmonary disease in an animal or human, comprising administering to the animal or human a pharmaceutical composition comprising a therapeutically effective amount of a compound of Formula (Ia).

In one embodiment, the pulmonary disease is asthma. In a particular embodiment, the asthma is allergic asthma.

In another embodiment, the pulmonary disease is chronic obstructive pulmonary disease (COPD).

In one embodiment, the compound of Formula (I) is (*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyridin-2-yl)propanamide, or a pharmaceutically acceptable salt thereof. In one embodiment, the pulmonary disease is asthma. In a particular embodiment, the asthma is allergic asthma. In another embodiment, the pulmonary disease is chronic obstructive pulmonary disease (COPD).

In yet another embodiment, the compound of Formula (Ia) is (*S*)-2-(1,3-dimethyl-2,6-dioxo-1,2,3,6-tetrahydro-7*H*-purin-7-yl)-*N*-(6-(2-((*S*)-2-methylpyrrolidin-1-yl)pyrimidin-5-yl)pyrazin-2-yl)propanamide, or a pharmaceutically acceptable salt thereof. In one embodiment, the pulmonary disease is asthma. In a particular embodiment, the asthma is allergic asthma. In another embodiment, the pulmonary disease is chronic obstructive pulmonary disease (COPD).

In still another embodiment, the compound of Formula (Ia) is (*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamide, or a pharmaceutically acceptable salt thereof. In one embodiment, the pulmonary disease is asthma. In a particular embodiment, the asthma is allergic asthma. In another embodiment, the pulmonary disease is chronic obstructive pulmonary disease (COPD).

The amount and concentration of compounds of Formula (Ia) in the pharmaceutical compositions, as well as the quantity of the pharmaceutical composition administered to a subject, can be selected based on clinically relevant factors, such as medically relevant characteristics of the subject (e.g., age, weight, gender, other medical conditions, and the like), the solubility of compounds in the pharmaceutical compositions, the potency and activity of the compounds, and the manner of administration of the pharmaceutical compositions. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

### Definitions

The terms "treat", "treated", "treating", or "treatment" includes the diminishment or alleviation of at least one symptom associated or caused by the state, disorder or disease being treated. For example, treatment can be diminishment of one or several symptoms of a disorder or complete eradication of a disorder.

As used herein, the term "modulating" or "modulate" refers to an effect of altering a biological activity, especially a biological activity associated with an injection site reaction.

The term "use" includes any one or more of the following embodiments of the invention, respectively: the use in the treatment of pain the use for the manufacture of pharmaceutical compositions for use in the treatment of these diseases, e.g., in the manufacture of a medicament; methods of use of compounds of the invention in the treatment of these diseases; pharmaceutical preparations having compounds of the invention for the treatment of these diseases; and compounds of the invention for use in the treatment of these diseases; as appropriate and expedient, if not stated otherwise.

The term "subject" is intended to include organisms, e.g., prokaryotes and eukaryotes, which are capable of suffering from or afflicted with a disease, disorder or condition associated with the activity of a protein kinase. Examples of subjects include mammals, e.g., humans, dogs, cows, horses, pigs, sheep, goats, cats, mice, rabbits, rats, and transgenic non-human animals. In certain embodiments, the subject is a human, e.g., a human suffering from, at risk of suffering from, or potentially capable of suffering from pain, respiratory conditions, and other TRPA1-related conditions described herein. In another embodiment, the subject is a cell.

As used herein, "substantially isolated" refers to a compound that is at least partially or substantially separated from the environment in which it was formed or detected. Partial separation can include, for example, a composition enriched in the compound of the invention. Substantial separation can include compositions containing at least about 50%, at least about 60%, at least about 70%, at least about 80%, at least about 90%, at least about 95%, at least about 97%, or at least about 99% by weight of the metabolite.

As used herein, "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the conventional non-toxic salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418 and Journal of Pharmaceutical Science, 66, 2 (1977).

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "therapeutically effective amount" indicates an amount that results in a desired pharmacological and/or physiological effect for the condition. The effect may be prophylactic in terms of completely or partially preventing a condition or symptom thereof and/or may be therapeutic in terms of a partial or complete cure for the condition and/or adverse effect attributable to the condition.

As used herein, the term "pharmaceutically acceptable carrier", and cognates thereof, refers to adjuvants, binders, diluents, etc. known to the skilled artisan that are suitable for administration to an individual (e.g., a mammal or non-mammal). Combinations of two or more carriers are also contemplated in the present invention. The pharmaceutically acceptable carrier(s) and any additional components, as described herein, should be compatible for use in the intended route of administration (e.g., oral, parenteral) for a particular dosage form. Such suitability will be easily recognized by the skilled artisan, particularly in view of the teaching provided herein. Pharmaceutical compositions described herein include at least one pharmaceutically acceptable carrier or excipient; preferably, such compositions include at least one carrier or excipient other than or in addition to water.

When used with respect to the use of the compounds and pharmaceutical compositions thereof described herein, an individual "in need thereof" may be an individual who has been diagnosed with or previously treated for the condition to be treated. With respect to prevention, the individual in need thereof may also be an individual who is at risk for a condition (e.g., a family history of the condition, life-style factors indicative of risk for the condition, etc.). Typically, when a step of administering a compound of the invention is disclosed herein, the invention further contemplates a step of identifying an individual or subject in need of the particular treatment to be administered or having the particular condition to be treated.

In some embodiments, the individual is a mammal, including, bovine, horse, feline, rabbit, canine, rodent, or primate. In some embodiments, the mammal is a primate. In some embodiments, the primate is a human. In some embodiments, the individual is human, including adults, children and premature infants. In some embodiments, the individual is a non-mammal. In some variations, the primate is a non-human primate such as chimpanzees and other apes and monkey species. In some embodiments, the mammal is a farm animal such as cattle, horses, sheep, goats, and swine; pets such as rabbits, dogs, and cats; laboratory animals including rodents, such as rats, mice, and guinea pigs; and the like. Examples of non-mammals include, but are not limited to, birds, and the like. The term "individual" does not denote a particular age or sex.

As used herein and in the appended claims, the singular forms "a", "an", and "the" include plural forms, unless the context clearly dictates otherwise.

Unless defined otherwise or clearly indicated by context, all technical and scientific terms used herein have the same meaning as commonly understood by one of ordinary skill in the art to which this invention belongs.

### EXAMPLES

### Example 1: Method for Measuring Inhibition of the TRPA1 Ion Channel

Compounds of Formula (Ia) inhibit the TRPA1 channel, as measured using the method of Example 1. Unless otherwise indicated, the method of Example 1 was used to measure *in vitro* inhibition of human TRPA1 provided in data tables shown in Figure 1, using the procedure outlined in del Camino et al., The Journal of Neuroscience, 30(45):15165-15174 (November 10, 2010), incorporated herein by reference and summarized below. Data for TRPA1 inhibition and for TRPA1 selectivity was obtained by this method for the indicated compounds of Formula (Ia), with the relevant data included in Tables 1a - f below. All currents were recorded in whole-cell configuration using EPC-9 and EPC-10 amplifiers and Patchmaster software (HEKA). Patch pipettes had a resistance of 1.5-3 MΩ and 60-75% of the series resistance was compensated. The standard pipette solution consisted of 140mM CsAsp, 10 mM EGTA, 10 mM HEPES, 2.27 mM MgCl₂, 1.91 mM CaCl₂, 4 mM MgATP, and 0.1-0.3 mM Na₂GTP, with pH adjusted to 7.2 with CsOH. In addition, a solution containing 145 mM CsCl, 10 mM HEPES, 10 mM EGTA and 1 mM MgCl₂ (pH 7.2 adjusted with CsOH) can be used. The standard bath solution contained 150 mM NaCl, 10 mM HEPES, 10 mM glucose, 4.5 mM KCl, 1 mM EGTA, 3 mM MgCl₂, with pH adjusted to 7.4 with NaOH. In some instances, 2 mM CaCl₂ was added in place of EGTA and the concentration of MgCl₂ was reduced to 1 mM.

Data were collected either by continuous recordings at -60 mV or by applying voltage ramps from a holding potential of 0 mV every 4 s. Continuous recordings were collected at 400 Hz and digitally filtered off-line at 10 Hz for presentation. Voltage ramps were applied from - 100 mV to 100 mV over the course of 400 ms, and data were collected at 10 kHz and filtered at 2.9 kHz. Inward and outward currents were analyzed from the ramps at -80 and 80 mV, respectively. Liquid junction potential correction was not used.

Solutions were switched using a gravity-fed continuous focal perfusion system. To achieve rapid temperature changes, two temperature control, and perfusion systems were employed simultaneously. For temperatures ≥ 22°C, a Warner Instruments bipolar temperature controller (TC-344B) and inline heater (SHM-8) were used. For temperatures below 22°C a Warner Instruments temperature controller (CL-100) and thermal cooling module (TCM-1) were used. Temperatures were confirmed using a thermistor (Warner Instruments, TA-29), with temperatures at the recorded cell estimated to be within +/- 2°C of those reported.

Tables 1a - f show data obtained from the *in vitro* assay described in Example 1, where an "A" value represents an IC₅₀ value of less than 25nM; a "B" value represents an IC₅₀ value between 25 and 75nM; a "C" value represent an IC₅₀ value between 76 and 100 nM; and a "D" value represents an IC₅₀ value exceeding 100nm. The antagonist effects of compounds of Formula (Ia) against human TRPA1 ("hTRPA1") and for some compounds rat TRPA1 ("rTRPA1") in a whole cell patch configuration were evaluated using the *in vitro* assay described above. The current activation tested was 10 µM AITC, and the tested concentrations ranged from 5 µM and 500 nM.

**Table 1a (Comparative Compounds)**

| **Compound No.** | **Compound Structure** | **Human TRPA1 IC₅₀ Inward Current (nM)** | **pH 4.0 aqueous solubility (mg/mL)** | **pH 7.4 aqueous solubility (mg/mL)** | **pH 9.0 aqueous solubility (mg/mL)** |
|---|---|---|---|---|---|
| Comparator 1 | 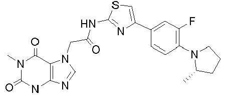 | A | | | |
| Comparator 2 | 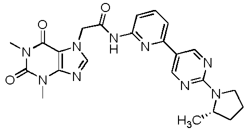 | C | 0.009 | 0.007 | 0.010 |
| Comparator 3 | 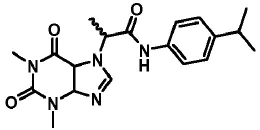 | D | | | |
| Comparator 4 | 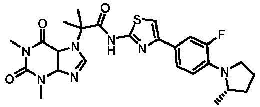 | D | | | |
| Comparator 5 | 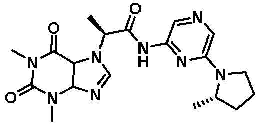 | D | | | |
| Comparator 6 | 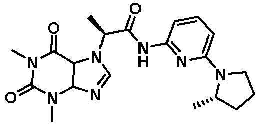 | D | | | |

**Table 1b**

| **Compound No.** | **Compound Structure** | **Human TRPA1 IC₅₀ Inward Current (nM)** | **pH 4.0 aqueous solubility (mg/mL)** | **pH 7.4 aqueous solubility (mg/mL)** | **pH 9.0 aqueous solubility (mg/mL)** |
|---|---|---|---|---|---|
| 1-A illustrative | 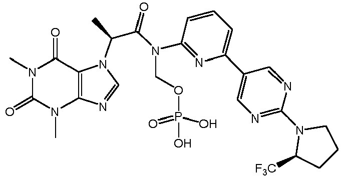 | D | > 1 | > 1 | > 1 |
| 1-B | 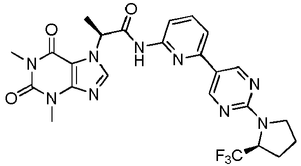 | A | 0.054 | 0.044 | 0.049 |
| 1-C ill ustrative | 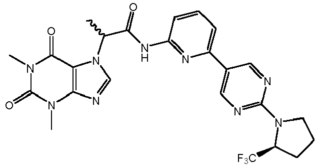 | A | | | |
| 1-D illustrative | 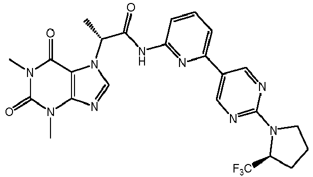 | D | | | |

**Table 1c**

| **Compound No.** | **Compound Structure** | **Human TRPA1 IC₅₀ Inward Current (nM)** | **pH 4.0 aqueous solubility (mg/mL)** | **pH 7.4 aqueous solubility (mg/mL)** | **pH 9.0 aqueous solubility (mg/mL)** |
|---|---|---|---|---|---|
| 5-S illustrative | 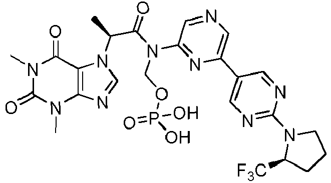 | D | > 1 | > 1 | > 1 |
| 5-T | 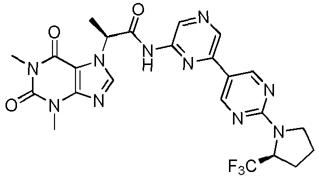 | A | 0.005 | 0.005 | 0.007 |
| 5-U illustrative | 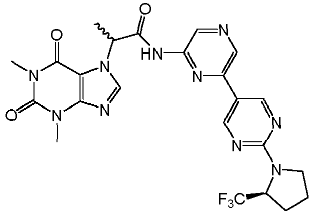 | A | | | |
| 5-V illustrative | 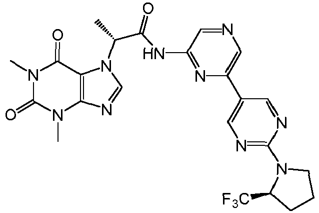 | D | | | |

**Table 1d**

| **Compound No.** | **Compound Structure** | **Human TRPA1 IC₅₀ Inward Current (nM)** | **pH 4.0 aqueous solubility (mg/mL)** | **pH 7.4 aqueous solubility (mg/mL)** | **pH 9.0 aqueous solubility (mg/mL)** |
|---|---|---|---|---|---|
| 2-E ill ustrative | 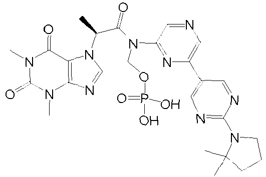 | D | > 1 | > 1 | > 1 |
| 2-F | 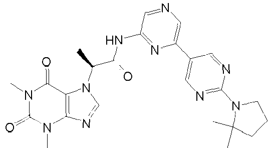 | A | 0.006 | 0.006 | 0.006 |
| 2-G illustrative | 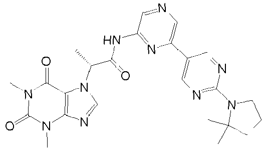 | D | | | |
| 2-H | 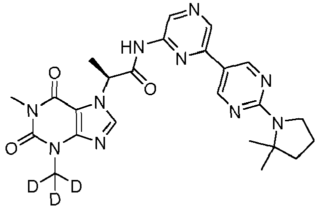 | A | | | |
| 2-I illustrative | 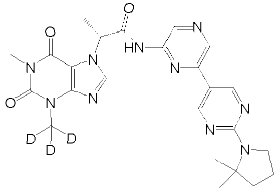 | D | | | |

**Table 1e**

| **Compound No.** | **Compound Structure** | **Human TRPA1 IC₅₀ Inward Current (nM)** | **pH 4.0 aqueous solubility (mg/mL)** | **pH 7.4 aqueous solubility (mg/mL)** | **pH 9.0 aqueous solubility (mg/mL)** |
|---|---|---|---|---|---|
| 3-J illustrative | 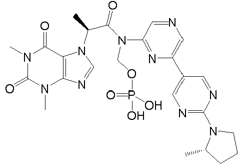 | D | > 1 | > 1 | > 1 |
| 3-K | 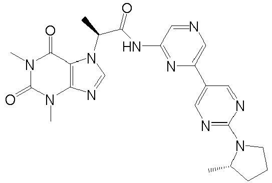 | B | 0.049 | 0.038 | 0.048 |
| 3-L illustrative | 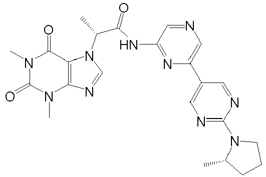 | D | | | |
| 3-M | 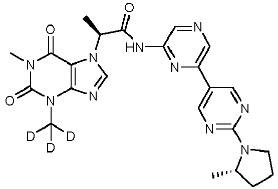 | A | | | |
| 3-N illustrative | 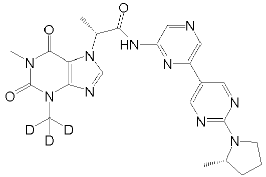 | D | | | |

**Table 1f**

| **Compound No.** | **Compound Structure** | **Human TRPA1 IC₅₀ Inward Current (nM)** |
|---|---|---|
| 4-P | 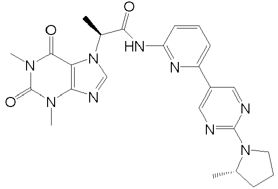 | B |
| 4-Q illustrative | 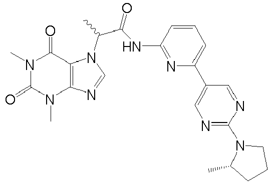 | B |
| 4-R illustrative | 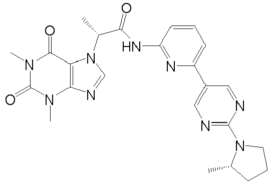 | D |

### Example 2: Method for Measuring Aqueous Solubility

Compounds of Formula (Ia) where R¹ is CH₂-R^{1a}, and R^{1a} is phosphate, have suitable levels of aqueous solubility for incorporation in a pharmaceutical composition formulated for intravenous administration. Unless otherwise indicated, the aqueous solubility of compounds of Formula (Ia) in Tables in Figure 1 was measured using the procedure of Example 2 (Kerns, E.H., Journal of Pharmaceutical Sciences 2001, 90, 1838-1858). Solubility data was obtained by this method for compounds of Formula (Ia) and included in Tables 1a - f. The chromatographic data was performed using (type of HPLC). The column used was an Xbridge Shield RP18 with the following column dimensions: 4.6 x 30mm, 3.5 µm. The mobile phase consisted of deionized water (MPA) with trifluoroacetic acid added in at 0.1% (v/v) (MPC) and HPLC-grade acetonitrile (MPB). The mobile phase flow rate was 2.5 mL/min with the column and sampling operating at ambient temperature. UV detection was set to 280 nm. For all samples used for solubility determination, the mobile phase gradient used was as follows in Table 1g below.

**Table 1g**

| **Time (min)** | **% MPA** | **% MPB** | **% MPC** |
|---|---|---|---|
| 0.00 | 70 | 20 | 10 |
| 1.08 | 0 | 90 | 10 |
| 1.20 | 0 | 90 | 10 |
| 1.21 | 70 | 20 | 10 |
| 1.50 | 70 | 20 | 10 |

Stock solutions of compounds of Formula (Ia) where R¹ is CH₂-R^{1a}, and R^{1a} is phosphate, were prepared in water at 20 mg/mL. Stock solutions of the compounds of Formula (Ia), wherein R¹ is hydrogen, were prepared in dimethyl acetamide at 20 mg/mL. All stock solutions of the compounds of Formula (Ia) were soluble at 20 mg/mL at ambient temperature.

Samples for analysis of the compounds of Formula (Ia) were prepared at a %v/v = 1/19 (i.e., 10 µL of the stock solution into 190 µL of buffer) by spiking stock solutions of the compounds of Formula (Ia) into buffered solutions. Three buffered solution systems were prepared: pH 4.0 prepared from 50mM sodium acetate in a 5% dextrose in water solution, pH 7.4 prepared from 75 mM sodium phosphate in a 1:1 ratio of sterilized water for injection to a 5% dextrose in water solution, and pH 9.0 prepared from 50 mM sodium bicarbonate in a 1:2 ratio of sterilized water for injection to a 5% dextrose in water solution.

The samples were incubated on a microplate shaker at 300 rpm for 24 hours at ambient temperature. Following incubation, the samples were centrifuged for five minutes at 13k rpm at ambient temperature. The resulting supernatant was extracted for HPLC analysis.

### Example 3: Rodent Formalin Pain Model

Compounds of Formula (Ia) are active in rodent models of pain induced by direct activation of the TRPA1 channel with intraplantar injection of the TRPA1 agonist formalin. Compounds of Formula (Ia) were tested in the formalin-induced pain test reported by Abbott et. al. Pain. 1995 Jan;60(1):91-102. Dubuisson et al. describe a method for assessing pain and analgesia in rats. Briefly, dilute formalin (50 µL of 3 % formalin) is injected into the plantar surface of the hind paw. The animal is promptly returned to an observation arena (standard Plexiglass rat cage), at which point a trained observer records the time the animal spends exhibiting pain behaviors (flinching, licking, biting of the injected paw/leg) for a period of 5 minutes (split out as the first 2 minutes and the total time period of five minutes). The individual responsible for counting the pain behaviors in a particular study is blinded to the treatment groups.

Table 2 is a data table showing the results from testing compounds of Formula (Ia) in the rodent formalin-induced pain model described in Example 2. Rats were treated with the compounds of Formula (Ia) at various doses (0.1, 0.3, 1, 1.2, 3, 4, 10, or 30 mg/Kg IV) or with a control composition containing the vehicle (i.e., without a compound of Formula (Ia)). Pain behaviors (flinch, lift, lick, bite) were monitored following intraplantar formalin injection. Results from the first two minutes are shown in Table 2. Referring to Table 2, the minimum effective dose was derived from dose resulting in statistically significant difference in behavior at 0 - 2 min time period as compared with vehicle (p≤0.05).

**Table 2**

| **Compound No.** | **Compound** | **Minimal Efficacious Dose of Compound in Formalin Model (mg/kg IV)** | **Dose (mg/kg)** | **Duration of Pain Behavior (sec)** | **Error (sec)** |
|---|---|---|---|---|---|
| Vehicle | | -- | -- | 88.6 | 4.3 |
| 1-A illustrative | 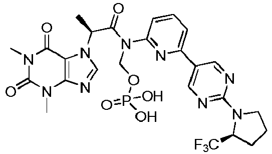 | 1.2 | 0.3 | 77.7 | 3.8 |
| | | | 1.2 | 43.3 | 8.4 |
| | | | 4 | 19.8 | 8 |
| 1-B | 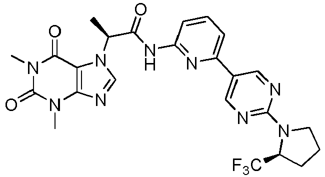 | <3 | 3 | 6.1 | 5.5 |
| | | | 10 | 0.3 | 0.3 |
| 2-E illustrative | 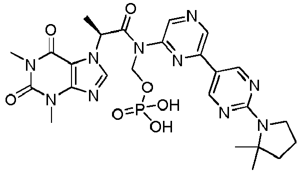 | 3 | 0.3 | 80.4 | 9.3 |
| | | | 1 | 77 | 10 |
| | | | 3 | 15.1 | 7.9 |
| 2-F | 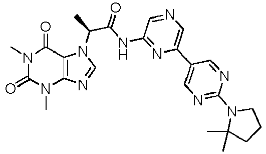 | 1 | 0.3 | 67.5 | 11 |
| | | | 1 | 7 | 4.4 |
| | | | 3 | 5 | 3 |
| 3-J illustrative | 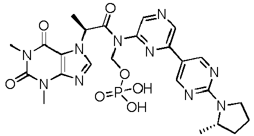 | 3 | 3 | 39.7 | 10.9 |
| | | | 10 | 1.6 | 1.1 |
| 3-K | 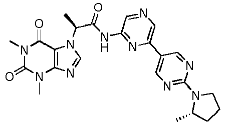 | <3 | 3 | 13.5 | 6.9 |
| | | | 10 | 2.6 | 1.4 |
| | | | 30 | 3.4 | 1.8 |
| 5-S illustrative | 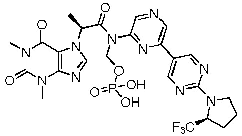 | 3 | 0.3 | 69.8 | 3.9 |
| | | | 1 | 52.3 | 13.8 |
| | | | 3 | 8.4 | 6.3 |
| | | | 10 | 0 | 0 |

### Example 4: Rodent Cold Plate Pain Model

The efficacy of compound of Formula (Ia) in treating pain was further demonstrated by the Complete Freund's Adjuvent (CFA) model of inflammatory pain in the rat. CFA-induced pain test method reported in del Camino et al., J. Neurosci., 30 (45):15165-15174. Briefly, the right hind paw is sensitized to cold temperature (allodynic) by administering 0.1 mL of Complete Freund's Adjuvant (CFA) to that paw. Two to four days later, the time it takes for the animal to lift its CFA-injected paw from the cold surface is recorded compared to its un-injected normal left hind paw. Animals are placed on the surface of the cold plate (1 °C) and the operator stops testing and notes the amount of time elapsed from first being placed on the plate to the instant the animal displays discomfort by flinching or lifting its paw from the plate (paw withdrawal latency, or PWL). To avoid tissue damage, the maximum cut-off time is five minutes. Animals that are allodynic (average PWL to the first three pain behaviors <150 seconds for the CFA-injected hind paw: ∼ ≥50% difference between the normal and CFA-injected paw) are included in the study and subsequently randomized across treatment groups. The following day, the animals in some groups are dosed with a test compound under blinded conditions. Following the one to two hour pre-treatment time, the post-dose PWL readings are taken again. The efficacy of the drug treatment is assessed by comparing the PWL in the drug treatment animals to those that receive the vehicle. The data obtained are shown in Figure 1 for tests using Compound 1-A, showing the reversal of CFA Cold Allodynia and therefore demonstrating the efficacy of the compound *in vivo.* In comparison, Figure 2 shows that Comparator 2 in the same model exhibiting significantly less potency.

### Example 5: Rodent Acute Tolerability Test

Compounds of Formula (Ia) were assessed for acute tolerability in a rodent model at dose levels ranging from 100 - 200 mg/kg based upon the minimum efficacious dose of each compound. Female Sprague Dawley rats were administered up to a 10 mL/kg dose volume of each compound in 0.9% saline at appropriate concentrations as an intravenous bolus via the lateral tail vein. The animals were observed immediately post dose and for up to twenty four hours post dose and clinical observations were recorded. Additionally, approximately twenty four hours post dose animals were euthanized and blood taken for serum chemistry analysis with a focus on liver function tests (LFTs). The maximum tolerated single dose and LFT results are summarized in Table 3, where (↔) indicates blood levels of markers for potential liver damage within normal range and (↑) indicates blood levels of markers for potential liver damage elevated above normal range. Referring to the data in Table 3, LFT blood levels of markers for potential liver damage remained within normal range for the compounds of Formula (Ia), while LFTs were elevated for Comparator Compounds including 5-10 fold elevation above normal for ALT and AST blood levels observed upon administration of Comparative Compound 1. Compounds of Formula (Ia) in Table 3 unexpectedly showed normal blood levels of the LFTs for ALT and AST in contrast to the elevated ALT and AST blood levels observed after about 24 hours after administration of the Comparative Compounds in Table 3.

**Table 3**

| **Compound** | **Compound** | **Single IV Bolus Dose (mg/kg)** | **Alterations in LFT** | **LFT: % upper normal range ALT/AST serum levels** |
|---|---|---|---|---|
| Comparator 1 | 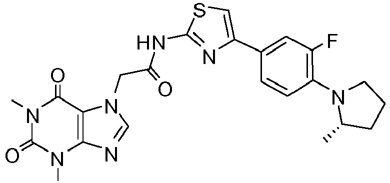 | 50 | ↑ | 1000%/514% |
| 1-A illustrative | 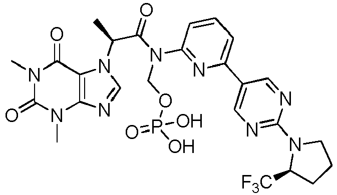 | 100 | ↔ | <100%/<100% |
| 2-E illustrative | 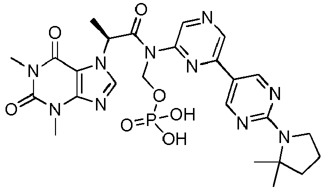 | 100 | ↔ | <100%/<100% |
| 3-J illustrative | 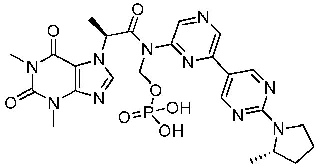 | 130 | ↔ | <100%/<100% |
| 5-S illustrative | 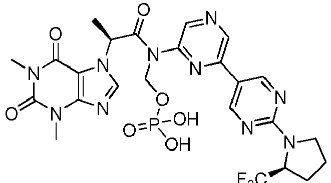 | 100 | ↔ | <100%/<100% |

### Example 6: Evaluation of Efficacy of a Compound of Formula (Ia) in an Allergic Asthma Model

### Animal Preparation

Compounds were tested in the sheep model of experimental asthma reported in Abraham WM, Asthma & Rhinitis, 2000: 1205-1227. All animals were tested for both early and late stage airway responses to inhalation challenge with *Ascaris suum* antigen. The sheep used for this study had previously been shown to develop early and late airway responses and airway hyperresponsiveness to inhaled carbachol following inhalation challenge with *Ascaris suum* antigen.

### Measurement of Airway Mechanics

The unsedated sheep were restrained in a cart in the prone position with their heads immobilized. After topical anesthesia of the nasal passages with 2% lidocaine solution, a balloon catheter was advanced through one nostril into the lower esophagus. The animals were intubated with a cuffed endotracheal tube through the other nostril. Pleural pressure was estimated with the esophageal balloon catheter. Lateral pressure in the trachea was measured with a sidehole catheter advanced through and positioned distal to the tip of the endotracheal tube. Transpulmonary pressure, the difference between tracheal and pleural pressure, was measured with a differential pressure transducer catheter system. For the measurement of pulmonary resistance (RL), the proximal end of the endotracheal tube was connected to a pneumotachograph. The signals of flow and transpulmonary pressure were recorded on an oscilloscope recorder, which was linked to a computer for on-line calculation of R_{L} from transpulmonary pressure, respiratory volume (obtained by digital integration) and flow. Analysis of 5-10 breaths were used for the determination of RL in cm H₂O / L x sec⁻¹.

### Aerosol Delivery Systems

Aerosols of Ascaris suum extract (diluted 20:1 with phosphate buffered saline; 82,000 PNU/ml) were generated using a disposable medical nebulizer (Raindrop^{R}, Puritan Bennett). The output from the nebulizer was directed into a plastic t-piece, one end of which was connected to the inspiratory port of a Harvard respirator. To better control aerosol delivery, a dosimeter consisting of a solenoid valve and a source of compressed air (20 psi) was activated at the beginning of the inspiratory cycle of the Harvard respirator system for 1 s. The aerosol was delivered at a tidal volume of 500 ml and a rate of 20 breaths per minute. Each sheep was challenged with an equivalent dose of antigen in the control and drug trials. Carbachol aerosols were also generated with this same nebulizer system.

### Concentration Response Curves to Inhaled Carbachol

For the carbachol concentration response curves, measurements of R_{L} were repeated immediately after inhalation of buffer and after each administration of 10 breaths of increasing concentrations of carbachol solution (0.25%, 0.5%, 1.0%, 2.0% and 4.0% w/v). To assess airway responsiveness, the cumulative carbachol dose in breath units (BU) that increased R_{L} 400% over the post-buffer value (i.e. PC 400) was calculated from the dose response curve. One breath unit was defined as one breath of a 1% w/v carbachol solution.

### Drug Treatment

Compound 5-T was given at doses of 1, 3 and 10 mg/kg given orally once a day for 3 days and on day 4, 2 hours before antigen challenge. A group of sheep also received a vehicle control.

### Experimental Protocol

A pre-challenge (baseline) PC 400 was obtained 1-3 days before the start of dosing. Then on the day of antigen challenge, the sheep received the last dose of Compound 5-T 2h before antigen challenge. Values of lung resistance (RL) were measured at baseline, 1.5 h after drug treatment and, then, serially for 8h after challenge with Ascaris suum antigen. Measurements of RL were obtained 24 h after challenge followed by the 24 h post challenge determination of PC 400. Responses to antigen challenge with drug and/or vehicle treatment were compared to each animal's historical control.

### Statistical Analysis

Two groups of animals (n=3) were used for these studies. Group 1 received the vehicle control and the 1 mg/kg dose. Group 2 received the 3 mg/kg dose and the 10 mg/kg dose. A Repeated Measures Analysis of Variance was used to compare the responses within each group to their historical control. If a significant difference was found, then Tukey's post hoc test was used to determine pair wise differences. The variables assessed were the average early airway response (EAR: average increase in RL 0-4h after challenge), average late airway response (LAR: average increase in RL between 5-8h after antigen challenge), and AHR (airway hyperresponsiveness). AHR was determined from the ratio of post challenge PC400 to pre challenge PC400. (Note: a ratio close to 1 indicates no airway hyperresponsiveness, whereas, a ratio close to 0.5 indicates the development of airway hyperresponsiveness). Values in the text and figures are mean ± se for 3 sheep. Significance was accepted when P<0.05.

### Results

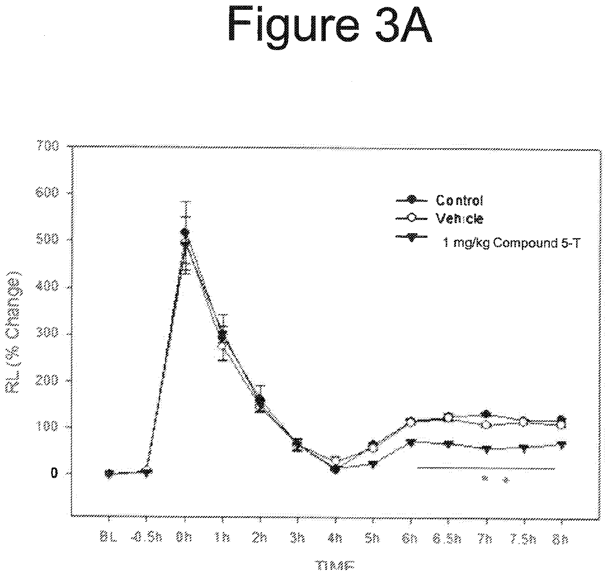

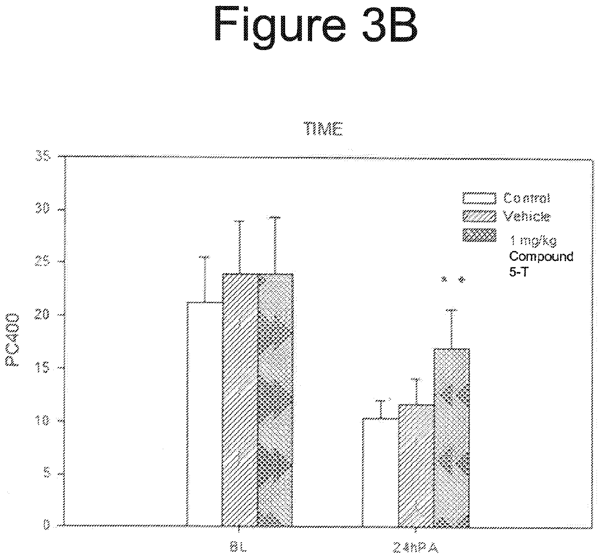

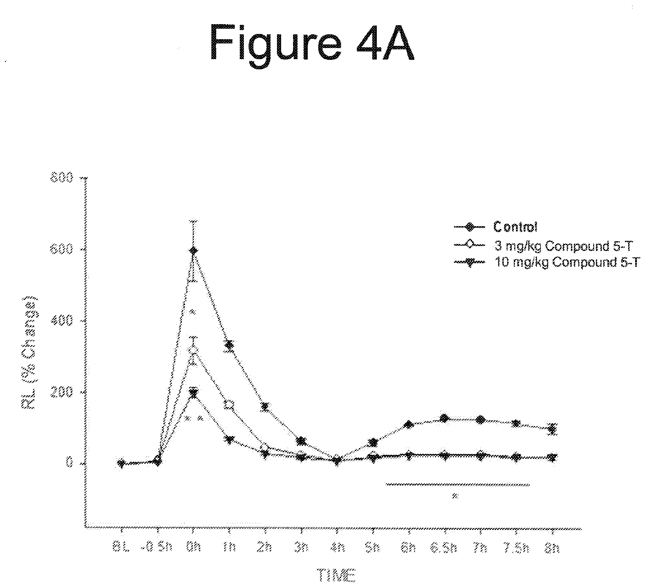

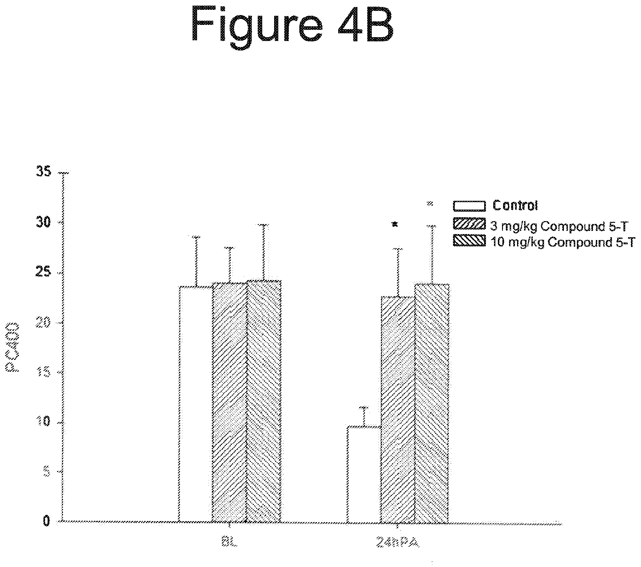

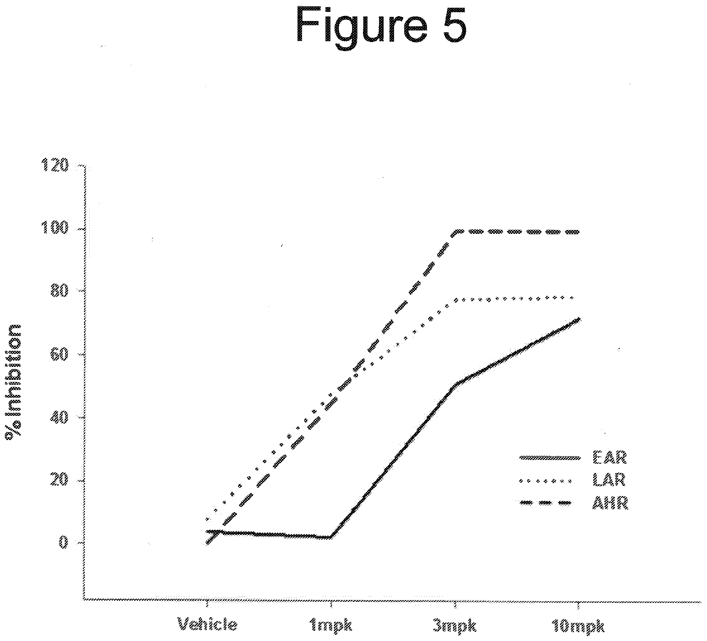

Figures 3A and 3B show the effect of the vehicle control and the 1mg/Kg dose of Compound 5-T and figures 4A and 4B show the effects of the 3 and 10 mg/Kg responses on antigen-induced responses in sheep compared to the animals' historical control response. Table 4 provides the statistical analysis for the results. The vehicle had no effect on any parameter (EAR, LAR or AHR). The 1 mg/Kg dose had no effect on EAR but significantly inhibited both the LAR (48%) and AHR (45%). Increasing the dose to 3 mg/Kg produced significant inhibition of the EAR (51%) as well as significant inhibition of the LAR (78%) and AHR (100%). Inhibition of the EAR was improved further by increasing the dose to 10 mg/Kg (72%). However, this dose was not different from the 3 mg/Kg dose in terms of protection against the LAR (79%) and AHR (100%). Figure 5 illustrates the inhibitory effect of the compound at the different doses on the three measured variables.

**Table 4. Effect of Compound 5-T on Antigen-Induced Responses**

| | **Average Early** | **Average Early** | **Airway** |
|---|---|---|---|
| | **Airway Response** | **Airway Response** | **Hyperresponsiveness** |
| | **(EAR)** | **(LAR)** | **(AHR)** |
| **Group 1** | | | |
| Control 1 | 175 ± 25 | 113 ± 4 | 0.49 ± 0.04 |
| Vehicle | 168 ± 12 | 104 ± 4 | 0.49 ± 0.02 |
| 1 mg/Kg of Compound 5-T | 171 ± 17 | 59 ± 1*⁺ | 0.71 ± 0.01*⁺ |
| | | | |

| **Group 2** | | | |
|---|---|---|---|
| Control 2 | 195 ± 15 | 107 ± 5 | 0.41 ± 0.01 |
| 3 mg/Kg of Compound 5-T | 95 ± 6* | 24 ± 1* | 0.92 ± 0.08* |
| 10 mg/Kg of Compound 5-T | 55 ± 2*^ | 22 ± 3* | 1.00 ± 0.14* |

### Example 7: TRPA1 Rodent Acute Tolerability Test

### Single-dose Toxicity Studies for Comparative Compound 2

In one single dose PK study conducted in fed and fasted rats, oral administration of Comparative Compound 2 in 30% HPCD produced adverse clinical signs in some of the fasted rats only. Specifically, signs of labored breathing and red urine were observed at dose levels of 50 mg/kg and 100 mg/kg for two of three and one of three fasted female rats, respectively,

In a preliminary single dose efficacy study, mild to moderate lethargy was observed in rats approximately 30 minutes to 4 hours following a single IP administration of Comparative Compound 2 at a dose level of 100 mg/Kg with an associated plasma Cₘₐₓ value of approximately 110 µg/mL.

Intravenous administration of Comparative Compound 2 (a nanosuspension formulation in 0.5% polysorbate 80 and 0.1% Poloxamer 188 and 0.8%NaCl) at dose levels of 3, 10, and 30 mg/kg as a single slow bolus (∼2 min) to male SD rat was well-tolerated. Mild flushing/mild swelling of the ears were observed at all three dose levels ∼1hr postdose and small red dotting in outer margin of pinna of ear (left ear only) was observed in seven of the nine animals. No abnormality of the ear was observed in histopathology.

### Single-Dose Toxicity Studies for Compound 1-B

Intravenous administration of Compound 1-B as a single, 30-minute infusion to SD female rats was well-tolerated at dose levels up to 300 mg/kg. No adverse clinical signs were observed concerning Compound 1-B.

### Single-dose Toxicity Studies for Compound 5-T

Intravenous administration of Compound 5-T as a single, 30-minute infusion to SD female rats was well-tolerated at dose levels up to 300 mg/kg. No adverse clinical signs were observed concerning Compound 5-T.

A single oral dose administration of Compound 5-T in a Spray Dried Dispersion (SDD) formulation (a suspension of SDD powder) at nominal doses of 3, 30, 100, and 300 mg/kg to fed female Sprague Dawley rats were well-tolerated. No adverse clinical signs were observed concerning Compound 5-T.

### Example 8: Synthesis of Intermediate Compounds

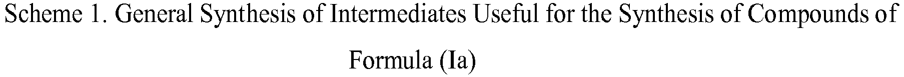

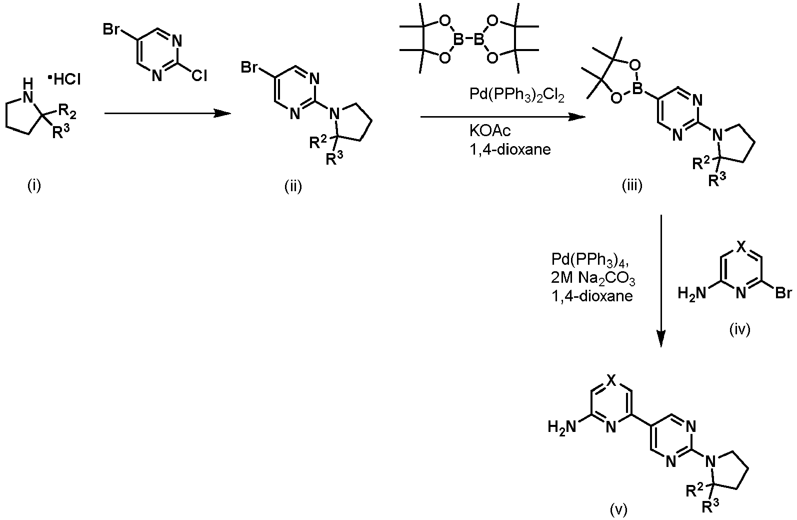

One or ordinary skill in the relevant technology can make compounds of Formula (Ia) based on the disclosure herein, including Example 9 and the Figures, using one or more intermediates disclosed and characterized in Example 8.

### Example 8A: Intermediate 1, (S)-5-Bromo-2-(2-(trifluoromethyl)pyrrolidin-1-yl)pyrimidine

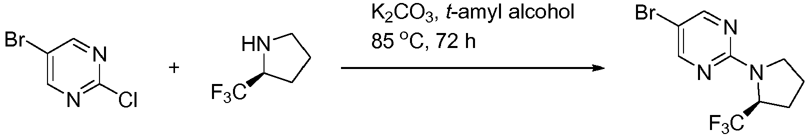

A flask was charged with 5-bromo-2-chloropyrimidine (36.62 g, 189 mmol), (S)-2-(trifluoromethyl)pyrrolidine (34.2 g, 246 mmol), potassium carbonate (39.2 g, 284 mmol)) and t-amyl alcohol (189 mL). The reaction mixture was stirred at 85 °C for 72 hours. The reaction mixture was then filtered over Celite®, washed with CH₂Cl₂ (2x) and concentrated *in vacuo.* The crude residue was redissolved in CH₂Cl₂ and washed with water and brine. The combined organic layers were dried over MgSO₄, filtered and concentrated *in vacuo* to give crystalline solids. The solids were washed with cold water (3x) and triturated with toluene to give the title product as pale orange crystals, 43.4 g (147 mmol, 77%); ¹H NMR (300 MHz, DMSO-d6) δ 8.58 (s, 2H), 5.06 - 4.83 (m, 1H), 3.71 - 3.44 (m, 2H), 2.30 - 1.89 (m, 4H); m/z found [M+H]⁺ = 295.99.

### Example 8B: Intermediate 2, (S)-5-(4,4,5,5-tetramethyl-1,3,2-dioxaborolan-2-yl)-2-(2-(trifluromethyl)pyrrolidin-1-yl)pyrimidine

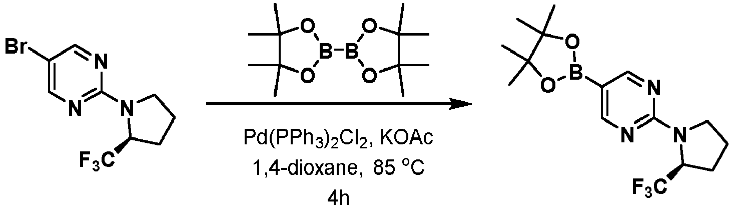

A dry flask was charged with compound (*S*)-5-bromo-2-(2-(trifluoromethyl)pyrrolidin-1-yl)pyrimidine (Intermediate 1, 43.4 g, 147 mmol), bis(pinacolato)diboron (44.7 g, 176 mmol), potassium acetate (28.8 g, 293 mmol) and bis(triphenylphosphine)palladium(II) chloride (10.29 g, 14.66 mmol). The flask was capped and purged with nitrogen (3x). The solids were suspended in anhydrous 1,4-dioxane (489 mL), stirred at 25 °C for 5 minutes and degassed with nitrogen for 15 minutes before stirring at 85 °C for 4 hours. The solvent was removed in vacuo. The resulting dark brown residue was suspended in water and extracted with CH₂Cl₂:MeOH 9:1 (3x). The combined organic layers were dried over MgSO₄, filtered and concentrated in vacuo. The crude material was purified on silica gel chromatography eluting with EtOAc:Hex (30:70) to yield 39.9 g (116 mmol, 79 %) of the title product as white to yellow flaky solids; ¹H NMR (300 MHz, DMSO-*d₆*) δ 8.56 (s, 2H), 5.24 - 4.93 (m, 1H), 3.78 - 3.48 (m, 2H), 2.30 - 1.90 (m, 4H), 1.29 (s, 12H); *m*/*z* found [M+H]+ = 261.07 (boronic acid).

### Example 8C: Intermediate 3, (S)-6-(2-(2-(trifluoromethyl)pyrrolidin-1-yl)pyrimidin-5-yl)pyridine-2-amine

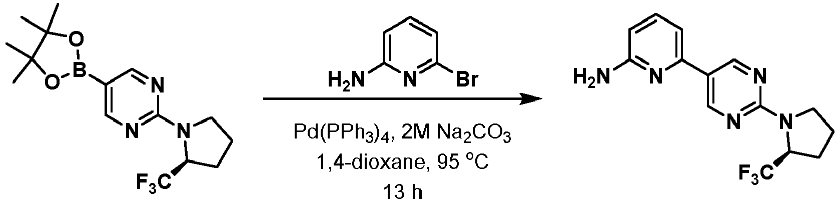

A flask was charged with (*S*)-5-(4,4,5,5-tetramethyl-1,3,2-dioxaborolan-2-yl)-2-(2-(trifluromethyl)pyrrolidin-1-yl)pyrimidine (Intermediate 2, 5 g, 14.57 mmol), 6-bromopyridin-2-amine (3.03 g, 17.48 mmol), Pd(PPh₃)₄ (1.684 g, 1.457 mmol) and 1,4-dioxane (36 mL). The reaction mixture was purged with nitrogen for several minutes before aqueous 2M sodium carbonate (14.57 mL, 29.1 mmol) solution was added. The reaction mixture was then purged with nitrogen for another 10 minutes at 25 °C before it was heated to 90 °C under nitrogen overnight. 1,4-Dioxane was removed in vacuo and the crude mixture was diluted with EtOAc. The product was washed with water (3x) and brine. The combined organic layers were dried over MgSO₄, filtered and concentrated under reduced pressure. The crude material was purified on silica gel chromatography eluting with MeOH:CH₂Cl₂ (2:98) to yield 3.75 g (12.13 mmol, 83 %) of the title product as an off white solid; ¹H NMR (300 MHz, DMSO-*d₆*) δ 8.99 (s, 2H), 7.44 (dd, *J=* 8.1, 7.5 Hz, 1H), 7.13 - 6.93 (m, 1H), 6.47 - 6.34 (m, 1H), 6.04 (s, 2H), 5.22 - 4.90 (m, 1H), 3.67 (t, *J=* 6.1 Hz, 2H), 2.32 - 1.95 (m, 4H); *m*/*z* found [M+H]⁺ = 310.45.

### Example 8D: Intermediate 4, (S)-N-((methylthio)methyl)-6-(2-(2-(trifluoromethyl)pyrrolidin-1-yl)pyrimidin-5-yl)pyridine-2-amine

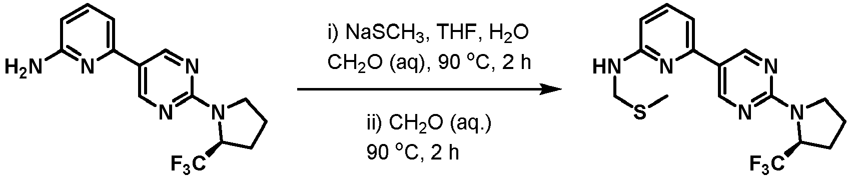

A 350 mL pressure vessel was charged with (*S*)-6-(2-(2-(trifluoromethyl)pyrrolidin-1-yl)pyrimidin-5-yl)pyridine-2-amine (Intermediate 3, 10 g, 32.3 mmol) and sodium methanethiolate (4.53 g, 64.7 mmol), which were dissolved in a mixture of THF (64.7 mL) and water (43.1 mL). Aqueous 37% formaldehyde (5.25 mL, 64.7 mmol) was added and the reaction mixture was heated to 90 °C for 2 hours. Another 2 eq. of aqueous 37% formaldehyde were added and the reaction mixture was heated for an additional 2 hours. The reaction mixture was then cooled to 25 °C. The layers were separated in the separatory funnel and the aqueous layer was extracted with EtOAc (3x). The combined organic layers were washed with brine, dried over MgSO4, filtered and concentrated in vacuo. The crude material was purified using silica gel chromatography, eluting with EtOAc:Hex (30:70) to yield 9 g (24.36 mmol, 75 %) of the title product as an oil; ¹H NMR (300 MHz, DMSO-*d₆*) δ 9.07 (s, 2H), 7.56 - 7.43 (m, 1H), 7.41 (t, *J* = 6.6 Hz, 1H), 7.13 (d, *J* = 7.3 Hz, 1H), 6.51 (d, *J* = 8.2 Hz, 1H), 5.18 - 4.98 (m, 1H), 4.59 (d, *J* = 6.7 Hz, 2H), 3.69 (dd, *J* = 10.3, 4.9 Hz, 2H), 2.21-2.0 (m, 4H), 2.12 (s, 3H); *m*/*z* found [M+H]⁺ = 370.35.

### Example 8E: Intermediate 5, (S)-2-(1,3-dimethyl-2,6-dioxo-1,2,3,6-tetrahydro-7H-purin-7-yl)-N-((methylthio)methyl)-N-(6-(2-(2-(trifluoromethyl)pyrrolidin-1-yl)pyrimidin-5-yl)pyridin-2-yl)acetamide

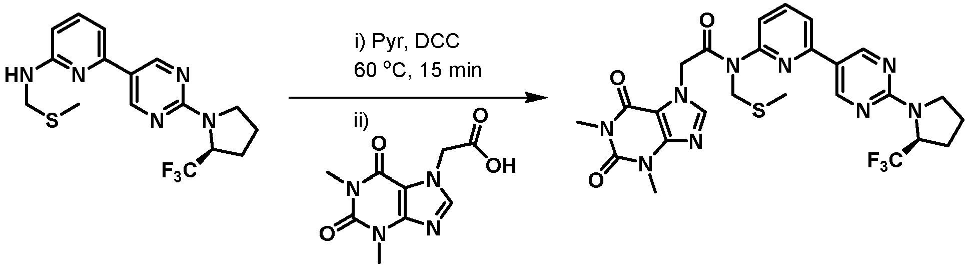

A solution of (*S*)-*N*-((methylthio)methyl)-6-(2-(2-(trifluoromethyl)pyrrolidin-1-yl)pyrimidin-5-yl)pyridine-2-amine (Intermediate 4,17 g, 46.0 mmol) and DCC (18.99 g, 92 mmol) in pyridine (230 mL) was heated for 30 minutes at 60 °C before theophylline 7-acetic acid (21.92 g, 92 mmol) was added in one portion. The reaction mixture was stirred at 60 °C and monitored by LCMS. After 2 hours, additional theophylline 7-acetic acid (21.92 g, 92 mmol) and DCC (18.99 g, 92 mmol) were added to drive the reaction to completion. The reaction mixture was diluted with CH₂Cl₂. The resulting precipitate (DCC urea) was filtered over Celite® and washed with CH₂Cl₂ (3x). The mixture was diluted with water and adjusted to pH 3 with aqueous 3M HCl. The organic and aqueous layers were separated and the aqueous layer was extracted with additional CH₂Cl₂ (3x). The combined organic layers were washed with aqueous 1M HCl (2x), dried over MgSO₄, filtered and concentrated *in vacuo.* The crude material was purified using silica gel chromatography, eluting with EtOAc:Hex (70:30) to yield 18.5 g (31.4 mmol, 68 %) of the title product as a thick oil; ¹H NMR (300 MHz, DMSO-*d₆*) δ 9.21 (s, 2H), 8.16 - 8.03 (m, 2H), 7.99 (d, *J* = 7.6 Hz, 1H), 7.60 (d, *J* = 7.7 Hz, 1H), 5.33 (s, 2H), 5.12 (m, 3H), 3.82 - 3.65 (m, 2H), 3.43 (s, 3H), 3.18 (s, 3H), 2.31 - 2.09 (m, 4H), 2.06 (s, 3H); *m*/*z* found [M+H]⁺ = 590.37.

Alternatively, the coupling of (*S*)-*N*-((methylthio)methyl)-6-(2-(2-(trifluoromethyl)pyrrolidin-1-yl)pyrimidin-5-yl)pyridine-2-amine and theophylline 7-acetic acid may be facilitated with propanephosphonic anhydride and DIPEA in acetonitrile at room temperature.

### Example 8F: Intermediate 6, (S)-2-(1,3-dimethyl-2,6-dioxo-1,2,3,6-tetrahydro-7H-purin-7-yl)-N-((methylthio(methyl)-N-(6-(2-((S)-2-(trifluoromethyl)pyrrolidin-1-yl)pyrimidin-5-yl)pyridin-2-yl)propanamide

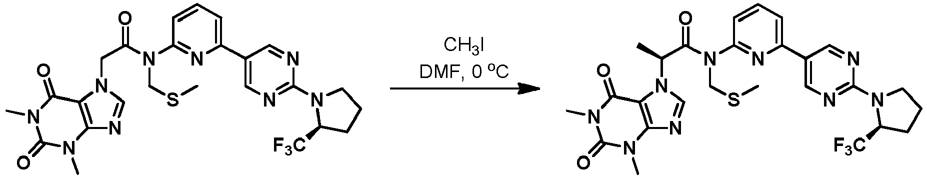

A solution of (*S*)-2-(1,3-dimethyl-2,6-dioxo-1,2,3,6-tetrahydro-7*H*-purin-7-yl)-*N-*((methylthio)methyl)-*N*-(6-(2-(2-(trifluoromethyl)pyrrolidin-1-yl)pyrimidin-5-yl)pyridin-2-yl)acetamide (Intermediate 5, 6.2 g, 10.52 mmol) in DMF (52.6 mL) was charged with iodomethane (1.312 mL, 21.03 mmol). The reaction mixture was cooled to 0 °C and 2-methyl-*N*-(tri(pyrrolidin-1-yl)phosphoranylidene)propan-2-amine (6.44 mL, 21.03 mmol) was added dropwise. The reaction mixture was stirred for 30 minutes. An additional 2 eq of iodomethane (1.312 mL, 21.03 mmol) and 2 eq of phosphazene base (6.44 mL, 21.03 mmol) were added to drive the reaction to completion. The reaction mixture was cooled to 0 °C and the pH was adjusted to 4-5 with aqueous 1M HCl. The reaction mixture was diluted with water and extracted with EtOAc (3x). The combined organic layers were dried over MgSO₄, filtered and concentrated in vacuo. The crude mixture of diastereomers material was purified via silica gel chromatography, eluting with MeOH:CH₂Cl₂ (2:98) to yield 14.2 g (23.52 mmol, 75 %) the title product as a yellow solid. A mixture of diastereomers was resolved using SFC purification to give the (*S*,*S*)-isomer in 6.21 g (retention time: 1.8 minutes, de: 98.5%) and the (*R*,*S*)-isomer in 6.72 g (retention time: 2.6 minutes, de: 96%); ¹H NMR, (*S*,*S*)-isomer: (300 MHz, DMSO-*d₆*) δ 8.87 (s, 2H), 8.12 (s, 1H), 8.04 (t, *J* = 7.8 Hz, 1H), 7.93 (d, *J=* 7.5 Hz, 1H), 7.59 (d, *J* = 7.6 Hz, 1H), 5.79 - 5.64 (m, 1H), 5.14 (d, *J* = 13.9 Hz, 1H), 5.09 - 5.00 (m, 1H), 4.92 (d, *J* = 13.9 Hz, 1H), 3.85 - 3.63 (m, 2H), 3.36 (s, 3H), 2.98 (s, 3H), 2.34 - 2.07 (m, 4H), 2.03 (s, 3H), 1.58 (d, *J* = 6.9 Hz, 3H); *m*/*z* found [M+H]⁺ = 604.49.

### Example 8G: Intermediate 7, Methyl (S)-2-(1,3-dimethyl-2,6-dioxo-1,2,3,6-tetrahydro-7H-purin-7-yl)propanoate

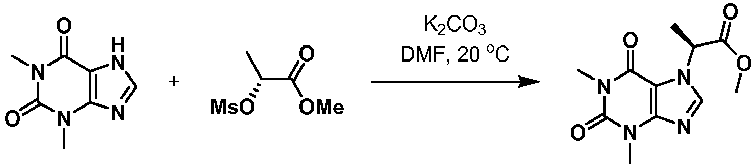

To a suspension of 1,3-dimethyl-1*H*-purine-2,6(3*H*,7*H*)-dione (20 g, 111 mmol) and K2CO3 (30.6 g, 222 mmol) in DMF (400 mL) was added (*R*)-methyl 2-((methylsulfonyl)oxy)propanoate (which may be synthesized according to the procedure described in PCT Pub No. WO/2005/059107 (40 g, 222 mmol). The reaction mixture was stirred at 20 °C for 20 h. The reaction mixture was then quenched with saturated NH₄Cl (aq), diluted with water and extracted with CH₂Cl₂ (3x). The combined organic extracts were washed 3x with 10% LiCl (aq), dried over Na₂SO₄ and evaporated in vacuo. The residue was purified using silica gel column chromatography eluting first with PE:CH₂Cl₂ (1:1) followed by CH₂Cl₂:MeOH (50:1) to give 13.5g (50.7 mmol, 45.7%, ee: 95%) of the (*S*)-product. The (*S*)-product was analyzed by chiral HPLC to determine it is at least 95% pure as the (*S*)-isomer (≥95% *ee*).

### Example 8H: Intermediate 8, (S)-2-(1,3-Dimethyl-2,6-dioxo-1,2,3,6-tetrahydro-7H-purin-7-yl)propanoic acid

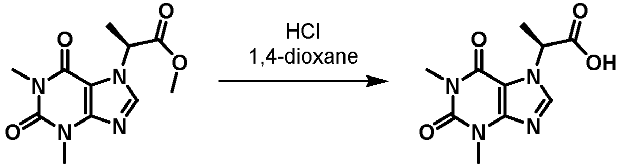

To a solution of methyl (*S*)-2-(1,3-dimethyl-2,6-dioxo-1,2,3,6-tetrahydro-7*H*-purin-7-yl)propanoate (Intermediate 7, 39 g, 146 mmol) in 1,4-dioxane (400 mL) was added 6N HCl (200 mL). The resulting mixture was refluxed for 3h, then cooled and concentrated under vacuum (water bath temp: 40 - 45 °C). Water (35 mL) was added to the residue, and the resulting mixture was then stirred for 0.5 h. The precipitate was collected by filtration, washed with cooled water and ethyl acetate to give the (*S*)-acid (17.3 g, ee: 99%). The filtrate was carefully neutralized with K₂HPO₄ solution (aq) to pH 4, extracted with (9:1) CH₂Cl₂:MeOH mixture (2x), dried over MgSO₄, filtered, and concentrated. The residue was purified using silica gel chromatography eluting with CH₂Cl₂:MeOH (9:1) to give additional (*S*)-acid (3.2 g, *ee*: 95%).

### Example 8I: Intermediate 9, 2-(1,3-Dimethyl-2,6-dioxo-1,2,3,6-tetrahydro-7H-purin-7-yl)-N-(6-(2-(2,2-dimethylpyrrolidin-1-yl)pyrimidin-5-yl)pyrazin-2-yl)-N-((methylthio)methyl)acetamide

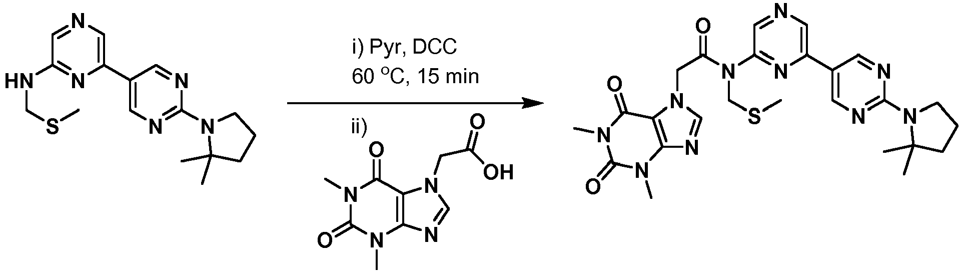

The title product was prepared from 6-(2-(2,2-dimethylpyrrolidin-1-yl)pyrimidin-5-yl)-N-((methylthio)methyl)pyrazin-2-amine (20.0 g, 60.5 mmol) using a procedure analogous to the one described for the synthesis of Intermediate 5. Yield: 21 g (33.9 mmol, 56%); ¹H NMR (300 MHz, DMSO-*d₆*) δ 9.17 (s, 1H), 9.14 (s, 2H), 8.82 (s, 1H), 8.08 (s, 1H), 5.41 (s, 2H), 5.19 (s, 2H), 3.68 (t, *J* = 6.5 Hz, 2H), 3.43 (s, 3H), 3.19 (s, 3H), 2.08 (s, 3H), 1.91 (dt, *J* = 12.2, 5.8 Hz, 4H), 1.54 (s, 6H); *m*/*z* [M+H]⁺ = 551.64.

### Example 9: Synthesis of Compounds of Formula (I) using Intermediates from Example 6

The following examples illustrate the synthesis of a composition comprising compounds of Formula (Ia), and pharmaceutically acceptable salts thereof. Further, the disclosure includes variations of the methods described herein to produce compounds of Formula (Ia) that would be understood by one skilled in the art. Scheme 1 is a general synthesis for intermediates useful in the synthesis of compounds of Formula (Ia), where X is N or CH. Scheme 2 is a general synthesis for compounds of Formula (Ia), wherein R¹ is hydrogen and X is N or CH. Scheme 3 is a general synthesis for compounds of Formula (Ia), wherein R1 is CH2-phosphate. In Scheme 4, X can be N or CH, R² can be H or CH₃, R³ can be CH₃ or CF₃ and R⁴ is as disclosed herein. Scheme 4 depicts a general procedure for preparing HCl salts for compounds of Formula (Ia) wherein R¹ is hydrogen. In Scheme 4, X can be N or CH, R² can be H or CH₃, while R³ can be CH₃ or CF₃.

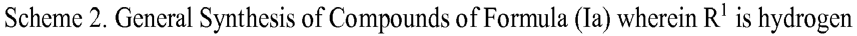

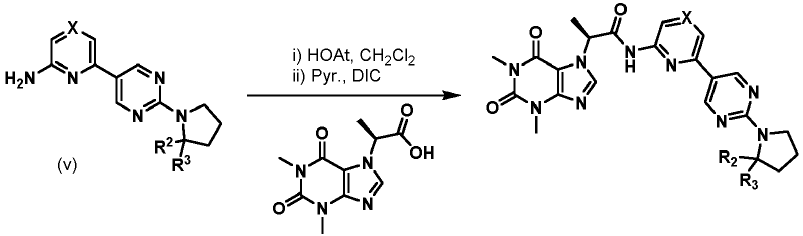

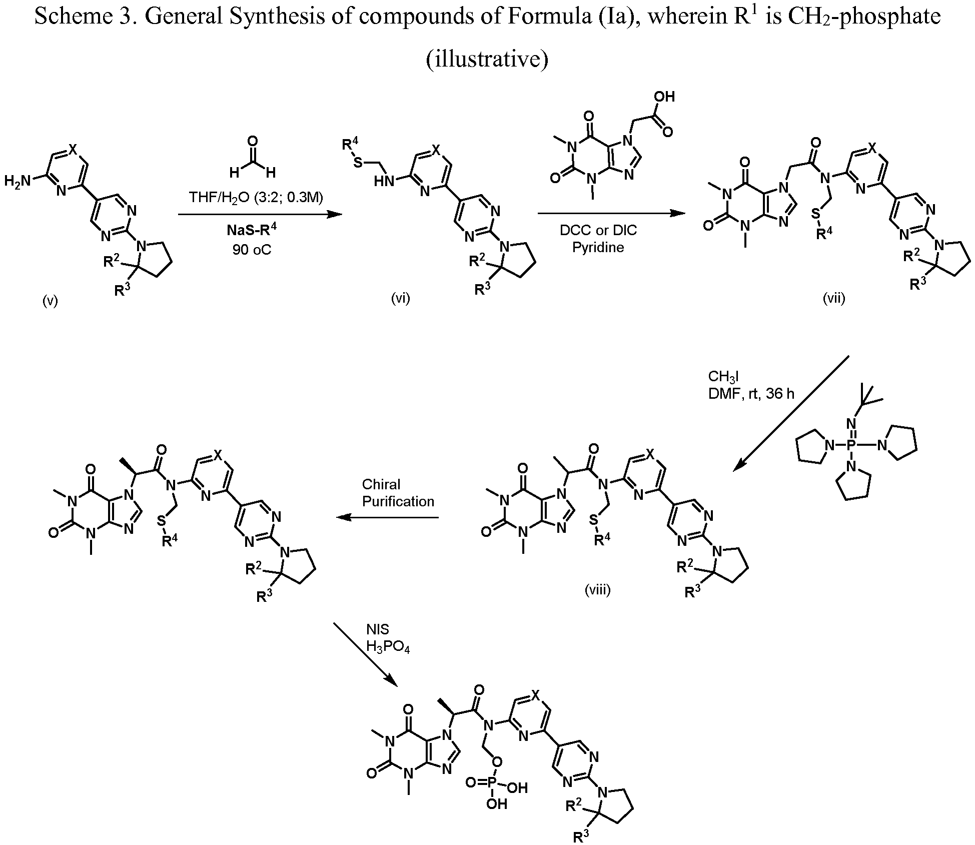

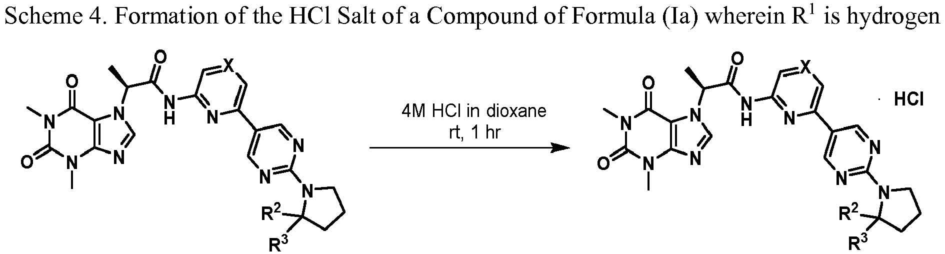

### Example 9A: Synthesis of ((S)-2-(1,3-dimethyl-2,6-dioxo-1,2,3,6-tetrahydro-7H-purin-7-yl)-N-(6-(2-((S)-2-(trifluoromethyl)pyrrolidin-1-yl)pyridine-2-yl)propanamido)methyl dihydrogen phosphate, sodium salt (Compound 1-A- illustrative)

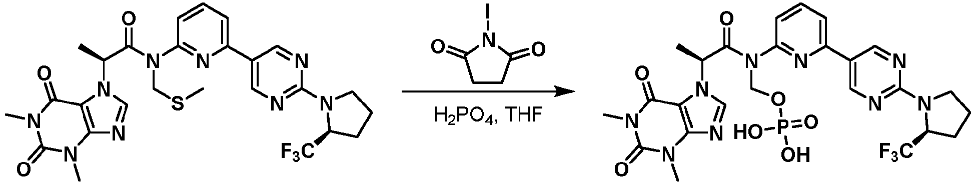

A dry flask was charged with (*S*)-2-(1,3-dimethyl-2,6-dioxo-1,2,3,6-tetrahydro-7*H*-purin-7-yl)-*N*-((methylthio(methyl)-*N*-(6-(2-((S)-2-(trifluoromethyl)pyrrolidin-1-yl)pyrimidin-5-yl)pyridin-2-yl)propanamide (Intermediate 6, 2.08 g, 3.45 mmol, de: 98.5%)) and 3Å activated molecular sieves (0.050 g). The flask was purged with nitrogen before crystalline phosphoric acid (9.79 g, 100 mmol) and anhydrous THF (17.23 mL) were added. The reaction mixture was stirred for 5 min at 25 °C before *N*-iodosuccinimide (NIS) (1.551 g, 6.89 mmol) was added in one portion. The reaction mixture was diluted with methanol and filtered over Celite®. The filtrate was cooled to 0 °C and quenched with saturated aqueous sodium thiosulfate followed by slow addition of aqueous NaOH (2 M), in 3 portions, to adjust to pH 7. The organic solvent was removed in vacuo at 25 °C and the aqueous solution was desalted over a Waters XBridge® C18 column and eluted with 60% acetonitrile. The lyophilized powder was then purified by prep-HPLC over a Waters XBridge® C18 column and eluted with 27% acetonitrile in 54 mM sodium phosphate buffer (pH 7.0). After removal of the organic solvent *in vacuo,* the desired fraction was further desalted and lyophilized to obtain 1.5 g (2.30 mmol, 66%, de > 95%) of the (*S*,*S*) product; ¹H NMR (300 MHz, DMSO-*d₆*) δ 8.87 (s, 2H), 8.23 (s, 1H), 7.93 (t, J = 7.9 Hz, 1H), 7.82 (d, *J=* 7.5 Hz, 1H), 7.57 (d, *J=* 7.9 Hz, 1H), 6.00 (d, *J=* 7.1 Hz, 1H), 5.59 (s, 1H), 5.41 (s, 1H), 5.14 - 4.98 (m, 1H), 3.79 - 3.61 (m, 2H), 3.40 (s, 3H), 2.98 (s, 3H), 2.33 - 1.99 (m, 4H), 1.67 (d, *J=* 6.4 Hz, 3H); *m*/*z* found [M+H]⁺ = 654.47. EA: C (40.07%), H (3.95%), N (16.65%), P (3.95%), Na (4.02%).

### Example 9B: Synthesis of (S)-2-(1,3-Dimethyl-2,6-dioxo-1,2,3,6-tetrahydro-7H-purin-7-yl)-N-(6-(2-((S)-2-(trifluoromethyl)pyrrolidin-1-yl)pyrimidin-5-yl)pyridin-2-yl)propanamide (Compound 1-B)

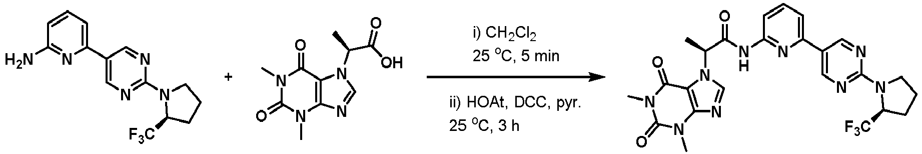

A flask was charged with (*S*)-6-(2-(2-(trifluoromethyl)pyrrolidin-1-yl)pyrimidin-5-yl)pyridine-2-amine (Intermediate 3, 5.44 g, 17.59 mmol) and (*S*)-2-(1,3-dimethyl-2,6-dioxo-1,2,3,6-tetrahydro-7*H*-purin-7-yl)propanoic acid (Intermediate 8, 5.10 g, 20.23 mmol, ee: 99%), CH2Cl2 (58.6 mL) and the resulting mixture was stirred at 25 °C for 5 minutes. To the resulting yellow heterogeneous mixture was added HOAt (2.394 g, 17.59 mmol) and DCC (6.90 g, 33.4 mmol). The reaction mixture was cooled to 0 °C and pyridine (2.85 mL, 35.2 mmol) was added slowly around the edges of the flask. The reaction mixture was kept at 0 °C for 5 minutes before it was removed from the ice bath, and was then stirred at 25 °C for 3 hours. The reaction mixture was diluted with CH₂Cl₂ and filtered over Celite® to remove urea byproduct. The filtrate was then quickly concentrated to an oil, taken up in minimal CH₂Cl₂ and added dropwise to stirring hexanes to precipitate out crude (*S*,*S*)-product which was dried overnight. The product was recrystallized from CH2Cl2:MeOH (4:1) to obtain the title product (4.78 g, 8.79 mmol, 50%, de > 99%); ¹H NMR (300 MHz, DMSO-*d₆*) δ 11.05 (s, 1H), 9.12 (s, 2H), 8.34 (s, 1H), 7.88 (dt, *J* = 15.7, 8.1 Hz, 2H), 7.68 (d, *J* = 8.2 Hz, 1H), 5.84 (d, *J* = 6.7 Hz, 1H), 5.11 (p, *J* = 7.4, 7.0 Hz, 1H), 3.83 - 3.61 (m, 2H), 3.46 (s, 3H), 3.19 (s, 3H), 2.33 - 1.96 (m, 4H), 1.87 (d, *J* = 7.3 Hz, 3H); *m*/*z* [M+H]⁺ = 544.20.

The compound of Example 9B was also synthesized using propane phosphonic acid anhydride (T₃P) as the coupling reagent, as shown in the following scheme:

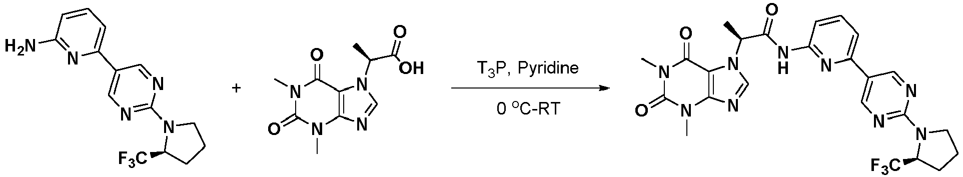

(*S*)-6-(2-(2-(trifluoromethyl)pyrrolidin-1-yl)pyrimidin-5-yl)pyridine-2-amine (Intermediate 3, 30.0 g, 1.0 eq) and (*S*)-2-(1,3-dimethyl-2,6-dioxo-1,2,3,6-tetrahydro-7*H*-purin-7-yl)propanoic acid (Intermediate 8, 29.4 g, 1.2 eq, ee: >98%) were charged to a 2.0 L ChemGlass reactor equipped with a nitrogen inlet, a temperature probe and an addition funnel. Acetonitrile (550 mL) was then added into the reactor. The resulting slurry was stirred for 10 minutes, and then cooled to 0-5 oC. Pyridine (27.6 g, 3.6 eq) was added dropwise to the slurry over 10 minutes, and the slurry was then stirred for 15 minutes. T3P (123.0 g, 2.0 eq, 50% in acetonitrile) was added dropwise to the slurry over 25-30 minutes. The reaction mixture was then stirred at 0-2 oC for 2 hours, warmed to room temperature, and stirring was continued until the reaction was completed. The slurry was then cooled to 10 oC and kept at 10 oC for 30 minutes. The slurry was then filtered, and the solid residue was washed with cold acetonitrile (2-5 oC, 2x50mL) followed by methyl t-butyl ether (MTBE) (100mL), and dried to produce the compound of Example 7B (39.4 g, 75%, de > 99.8%).

The hydrochloride salt of Example 9B may be prepared according to the procedure detailed below:

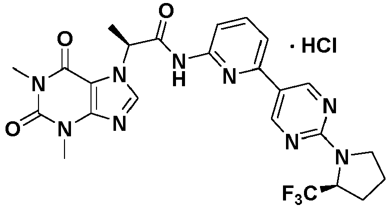

To a 1L round bottom flask charged with dry (*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5 - yl)pyridin-2-yl)propanamide (Example9B, 20.5 g, 43.1 mmol) was added 200 mL 4M HCl in dioxane. The resulting mixture was stirred at room temperature for 1 hr, during which time the reaction mixture change from a suspension, to a mostly homogenous clear yellow solution, to a white solid suspension in light yellow solvent. After 1 hr, the solids were collected via vacuum filtration with EtOH. The solids were rinsed with EtOH (3 x 100 mL) and placed under a high vacuum overnight. After 18 hr the material was removed from the high vacuum and transferred to an amber jar, providing the title product 22.6 g (> 100%) as an off-white solid. (I) salt (*m*/*z* = M⁺ = 475), 1H NMR (300 MHz, DMSO-*d₆*) δ 11.06 (s, 1H), 9.12 (s, 2H), 8.34 (s, 1H), 7.88 (dt, *J* = 15.7, 8.2 Hz, 2H), 7.68 (d, *J* = 8.4 Hz, 1H), 5.84 (d, *J* = 7.0 Hz, 1H), 5.13 (q, *J* = 8.1 Hz, 1H), 3.84 - 3.60 (m, 2H), 3.46 (s, 3H), 3.19 (s, 3H), 2.32 - 1.95 (m, 4H), 1.87 (d, *J* = 7.3 Hz, 3H).

### Example 10: Synthesis of (S)-2-(1,3-dimethyl-2,6-dioxo-1,2,3,6-tetrahydro-7H-purin-7-yl)-N-(6-(2-((S-2-(trifluoromethyl)pyrrolidin-1-yl)pyrimidin-5-yl)pyrazin-2-yl)propanamide

### Example 10A: Synthesis of (S)-5-Bromo-2-(2-(trifluoromethyl)pyrrolidin-1-yl)pyrimidine

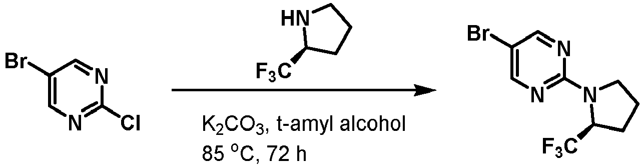

A flask was charged with 5-bromo-2-chloropyrimidine (36.62 g, 189 mmol), (S)-2-(trifluoromethyl)pyrrolidine (34.2 g, 246 mmol), potassium carbonate (39.2 g, 284 mmol)) and t-amyl alcohol (189 mL). The reaction mixture was stirred at 85 °C for 72 hours. The reaction mixture was then filtered over Celite®, washed with CH₂Cl₂ (2x) and concentrated in vacuo. The crude residue was redissolved in CH₂Cl₂ and washed with water and brine. The combined organic layers were dried over MgSO₄, filtered and concentrated in vacuo to give crystalline solids. The solids were washed with cold water (3x) and triturated with toluene to give the title product as pale orange crystals, 43.4 g (147 mmol, 77%); ¹H NMR (300 MHz, DMSO-*d₆*) δ 8.58 (s, 2H), 5.06 - 4.83 (m, 1H), 3.71 - 3.44 (m, 2H), 10 2.30 - 1.89 (m, 4H); *m*/*z* found [M+H]⁺ = 295.99.

### Example 10B: Synthesis of (S)-5-(4,4,5,5-tetramethyl-1,3,2-dioxaborolan-2-yl)-2-(2-(trifluromethyl)pyrrolidin-1-yl)pyrimidine

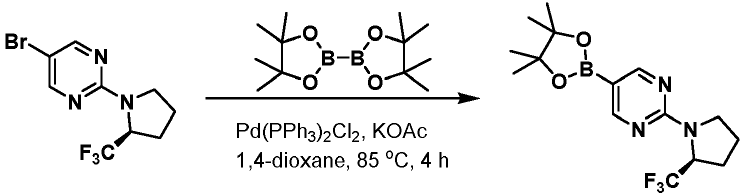

A dry flask was charged with compound (*S*)-5-bromo-2-(2-(trifluoromethyl)pyrrolidin-1-yl)pyrimidine (43.4 g, 147 mmol), bis(pinacolato)diboron (44.7 g, 176 mmol), potassium acetate (28.8 g, 293 mmol) and bis(triphenylphosphine) palladium(II) chloride (10.29 g, 14.66 mmol). The flask was capped and purged with nitrogen (3x). The solids were suspended in anhydrous 1,4-dioxane (489 mL), stirred at 25 °C for 5 minutes and degassed with nitrogen for 15 minutes before stirring at 85 °C for 4 hours. The solvent was removed in vacuo. The resulting dark brown residue was suspended in water and extracted with CH₂Cl₂:MeOH 9:1 (3x). The combined organic layers were dried over MgSO₄, filtered and concentrated in vacuo. The crude material was purified on silica gel chromatography eluting with EtOAc:Hex (30:70) to yield 39.9g (116 mmol, 79 %) of the title product as white to yellow flaky solids; ¹H NMR (300 MHz, DMSO-*d₆*) δ 8.56 (s, 2H), 5.24 - 4.93 (m, 1H), 3.78 - 3.48 (m, 2H), 2.30 - 1.90 (m, 4H), 1.29 (s, 12H); *m*/*z* found [M+H]⁺ = 261.07 (boronic acid)

### Example 10C: Synthesis of (S)-6-(2-(2-(trifluoromethyl)pyrrolidin-1-yl)pyrimidin-5-yl)pyrazin-2-amine

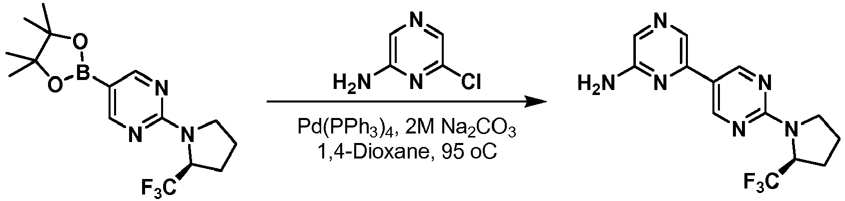

A round bottom flask was charged with 6-chloropyrazin-2-amine (0.401 g, 3.09 mmol), (*S*)-5-(4,4,5,5-tetramethyl-1,3,2-dioxaborolan-2-yl)-2-(2-(trifluoromethyl)pyrrolidin-1-yl)pyrimidine (1.062 g, 3.09 mmol), Pd(PPh₃)₄ (0.358 g, 0.309 mmol) and 1,4-Dioxane (7.74 mL). The reaction mixture was purged with nitrogen for several minutes before an aqueous 2M sodium carbonate (3.09 mL, 6.19 mmol) was added. The reaction mixture was purged for another 10 minutes under nitrogen before it was placed in a 90 °C oil bath and allowed to stir overnight. 1,4-Dioxane was removed *in vacuo* and the crude mixture was diluted with EtOAc. The product was wash with water (3x) and brine. The combined organic layers were dried over MgSO₄, filtered and concentrated under reduced pressure. The crude material was purified on silica gel using normal phase flash chromatography, eluting with CH₂Cl₂:MeOH to yield 0.655 g (2.11 mmol, 68.2%) of the title product; ¹H NMR (300 MHz, DMSO-*d₆*) δ 9.01 (s, 2H), 8.26 (s, 1H), 7.83 (s, 1H), 6.55 (s, 2H), 5.16 - 5.02 (m, 1H), 3.77 - 3.60 (m, 2H), 2.29 - 2.01 (m, 4H); *m*/*z* [M+H]⁺ = 311.12

### Example 10D: Synthesis of (S)-2-(1,3-dimethyl-2,6-dioxo-1,2,3,6-tetrahydro-7H-purin-7-yl)-N-(6-(2-((S)-2-(trifluoromethyl)pyrrolidin-1-yl)pyrimidin-5-yl)pyrazin-2-yl)propanamide (Compound 5-T)

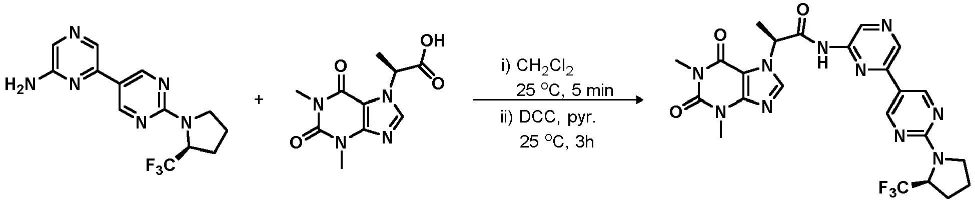

A round bottom flask was charged with (*S*)-6-(2-(2-(trifluoromethyl)pyrrolidin-1-yl)pyrimidin-5-yl)pyrazin-2-amine (5.097 g, 16.43 mmol) and (*S*)-2-(1,3-dimethyl-2,6-dioxo-2,3-dihydro-1*H*-purin-7(6H)-yl)propanoic acid (Intermediate 8) (4.97 g, 19.71 mmol). The reagents were suspended in DCM (36.5 mL), sonicated and allowed to stir for 5 minutes. To the yellow heterogenous mixture was added DCC (5.76 g, 27.9 mmol) and finally pyridine (Volume: 18.25 mL, Ratio: 2) was added and the mixture was allowed to stir for 3 hours. The reaction mixture was diluted with CH₂Cl₂ and filtered over Celite®. The filtrate was then concentrated and redissolved in a minimal amount of and added dropwise to stirring hexanes to precipitate out crude 2-(1,3-dimethyl-2,6-dioxo-2,3-dihydro-1*H*-purin-7(6*H*)-yl)-*N*-(6-(2-((*S*)-2-(trifluoromethyl)pyrrolidin-1-yl)pyrimidin-5-yl)pyrazin-2-yl)propanamide, which was dried overnight. The mixture of diasteromers was resolved using SFC purification to give the (*S*,*S*)-isomer in 2.58 g (retention time: 2.5 min, de: 98%); and the (*R*,*S*)-isomer in 0.208 g (retention time: 2.2 min, de: 99%); ¹H NMR, (S,S)-isomer (300 MHz, DMSO-*d₆*) δ 11.40 (s, 1H), 9.16 (d, *J* = 3.5 Hz, 3H), 8.97 (s, 1H), 8.36 (s, 1H), 5.86 (d, *J* = 7.3 Hz, 1H), 5.20 - 5.03 (m, 1H), 3.73 (t, *J* = 6.5 Hz, 2H), 3.46 (s, 3H), 3.19 (s, 3H), 2.29 - 2.04 (m, 4H), 1.89 (d, *J* = 7.3 Hz, 3H); *m*/*z* found [M+H]⁺ = 545.19

The compound of Example 10D was also synthesized using propane phosphonic acid anhydride (T₃P) as the coupling reagent as shown in the following scheme:

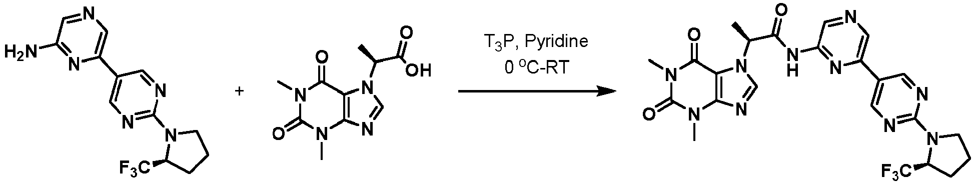

(*S*)-6-(2-(2-(trifluoromethyl)pyrrolidin-1-yl)pyrimidin-5-yl)pyrazin-2-amine (35.0 g, 1.0 eq) and (*S*)-2-(1,3-dimethyl-2,6-dioxo-2,3-dihydro-1*H*-purin-7(6*H*)-yl)propanoic acid (Intermediate 8, 34.1 g, 1.2 eq, *ee:* >98%) were charged to a 2.0 L ChemGlass reactor equipped with a nitrogen inlet, a temperature probe and an addition funnel. Dichloromethane (600 mL) was then added into the reactor. The resulting slurry was stirred for 10 minutes, and then cooled to 0-5 °C. Pyridine (32.1 g, 3.6 eq) was added dropwise to the slurry over 10 minutes, and the slurry was then stirred for 15 minutes. T₃P (144.0 g, 2.0 eq, 50% in dichloromethane) was then added dropwise to the slurry over 25-30 minutes. The reaction mixture was stirred at 0-5 °C for 4 hours, warmed to room temperature, and stirring was continued until the reaction was completed. Water (250 mL) was then added to wash the reaction mixture. After discarding the aqueous layer, the organic layer was washed with water (250 mL), aqueous KHCO₃ (200 mL, 1%), and aqueous brine (250 mL, 10% by weight). The organic layer was concentrated down, and acetone was then added and evaporated to remove residual dichloromethane. The resulting slurry was then transferred back to the flask. Acetone was added to adjust the volume of the slurry to 600 mL. The slurry was heated to reflux for 1 hour, and then cooled to room temperature over 2 hours and kept at room temperature for 30 minutes. The slurry was filtered, and the solid residue was washed with acetone (2x100mL) and dried to produce a crude product (50 g). The crude product was transferred to a 2-neck round bottom flask equipped with a mechanical stirrer and a nitrogen inlet. Acetone (500mL) was added into the flask. The resulting slurry was heated to reflux and then cooled to room temperature over 2 hours. The slurry was filtered, and the solid residue was washed with acetone (2 x 75 mL) and dried to produce the compound of Example 8D (43.0 g, 70%, de > 98%).

### Example 11: Synthesis of (S)-2-(1,3-dimethyl-2,6-dioxo-1,2,3,6-tetrahydro-7H-purin-7-yl)-N-(6-(2-((S)-2-methylpyrrolidin-1-yl)pyrimidin-5-yl)pyrazin-2-yl)propenamide (illustrative)

### Example 11A: Synthesis of (S)-5-bromo-2-(2-methylpyrrolidin-1-yl)pyrimidine

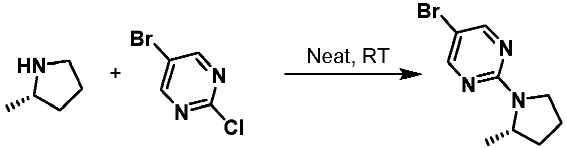

Solid 5-bromo-2-chloropyrimidine (27.5 g, 142 mmol) was added in three portions to a 500 mL flask containing (S)-2-methylpyrrolidine (20.24 mL, 213 mmol) stirred at room temperature. After 1 hr the resulting solids were dissolved in warm DCM, washed with water, brine, dried over MgSO₄ and concentrated onto silica *in vacuo.* Column purified by silica gel chromatography to afford white crystalline solids (30.7 g, 89%), ESI-MS (EI⁺, *m*/*z*): 241.02, 1H NMR (Chloroform-*d*): δ 8.31 (d, *J* = 5.0 Hz, 2H), 4.33 - 4.14 (m, 1H), 3.61 (tt, *J* = 7.6, 3.0 Hz, 1H), 3.56-3.41 (m, 1H), 2.23 - 1.88 (m, 3H), 1.88 - 1.63 (m, 1H), 1.25 (t, *J* = 5.5 Hz, 3H)

### Example 11B: Synthesis of (S)-2-(2-methylpyrrolidin-1-yl)-5-(4,4,5,5-tetramethyl-1,3,2-dioxaborolan-2-yl)pyrimidine

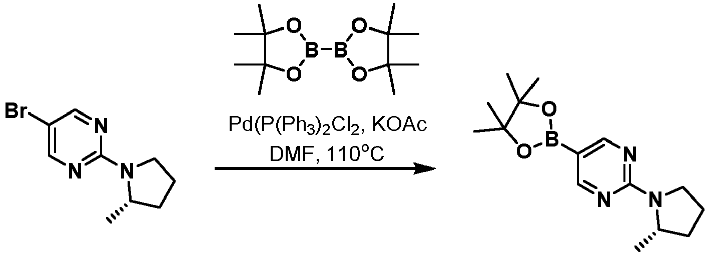

(*S*)-5-bromo-2-(2-methylpyrrolidin-1-yl)pyrimidine (18 g, 74.3 mmol), *bis*(pinacolato)diboron (28.3 g, 112 mmol), *bis*(triphenylphosphine)palladium chloride (10.44 g, 14.87 mmol) and potassium acetate (14.59 g, 149 mmol) were suspended in anhydrous dioxane (Volume: 372 mL). Nitrogen was bubbled through the solution for 15 minutes and then heated to reflux at 110 °C. After 2 hours, the reaction mixture was cooled to room temperature and the solvents were removed *in vacuo.* To the resulting residue was added water and extracted with DCM. The combined organic layers were washed with brine, dried over MgSO₄ and concentrated *in vacuo.* Column purified by silica gel chromatography to afford yellow solids, (14.3 g, 66%) ESI-MS (EI⁺, *m*/*z*): 289.2, ¹H NMR (DMSO-*d₆*) δ: 8.45 (s, 2H), 4.33 - 4.16 (m, 1H), 3.57 (ddd, *J* = 10.4, 7.5, 2.8 Hz, 1H), 3.51 - 3.38 (m, 1H), 2.13 - 1.82 (m, 3H), 1.75 - 1.61 (m, 1H), 1.27 (s, 12H), 1.17 (d, *J* = 6.3 Hz, 3H)

### Example 11C: Synthesis of (S)-6-(2-(2-methylpyrrolidin-1-yl)pyrimidin-5-yl)pyrazin-2-amine

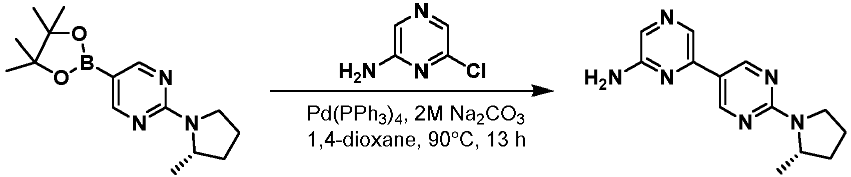

A flask was charged with (*S*)-2-(2-methylpyrrolidin-1-yl)-5-(4,4,5,5-tetramethyl-1,3,2-dioxaborolan-2-yl)pyrimidine (7.1 g, 24.55 mmol), 6-chloropyrazin-2-amine (2.89 g, 22.32 mmol), Pd(PPh₃)₄ (2.58 g, 2.232 mmol) and 1,4-dioxane (74.4 mL). The reaction mixture was purged with nitrogen for several minutes before aqueous 2M sodium carbonate (22.32 mL, 44.6 mmol) solution was added. The reaction mixture was then purged with nitrogen for another 10 minutes at 25 °C before it was heated to 90 °C under nitrogen overnight. 1,4-Dioxane was removed *in vacuo* and the crude mixture was diluted with EtOAc. The product was washed with water and brine. The combined organic layers were dried over MgSO₄, filtered and concentrated under reduced pressure. The crude material was purified on silica gel chromatography to yield 3.38 g (13.2 mmol, 84%) of the title product as a light brown solid. ESI-MS (EI⁺, *m*/*z*): 258.26, ¹H NMR (Chloroform-*d*) δ: 8.92 (d, *J* = 5.3 Hz, 2H), 8.25 (s, 1H), 7.88 (s, 1H), 4.60 (s, 2H), 4.39 (d, *J* = 5.8 Hz, 1H), 3.81 - 3.68 (m, 1H), 3.68 - 3.55 (m, 1H), 2.24 - 1.95 (m, 3H), 1.78 (s, 1H), 1.31 (d, *J* = 6.3 Hz, 3H)

### Example 11D: Synthesis of (S)-2-(1,3-dimethyl-2,6-dioxo-1,2,3,6-tetrahydro-7H-purin-7-yl)-N-(6-(2-((S)-2-methylpyrrolidin-1-yl)pyrimidin-5-yl)pyrazin-2-yl)propanamide (Compound 3-N-illustrative)

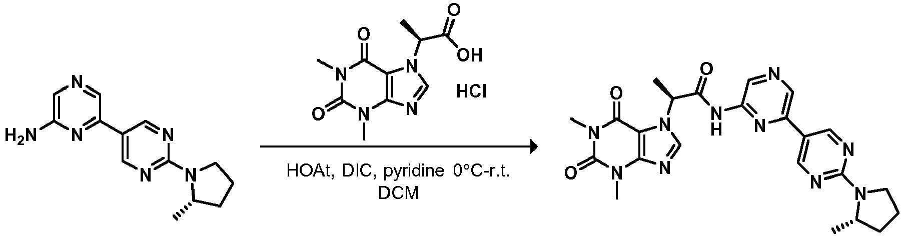

A flask was charged with (*S*)-2-(1,3-dimethyl-2,6-dioxo-2,3-dihydro-1*H*-purin-7(6*H*)-yl)propanoic acid hydrochloride (Intermediate 8) (8.96 g, 31.0 mmol), (*S*)-6-(2-(2-methylpyrrolidin-1-yl)pyrimidin-5-yl)pyrazin-2-amine (7.23 g, 28.2 mmol), HOAt (3.84 g, 28.2 mmol), nitrogen, DCM (141 mL) and stirred at room temperature. The suspension was cooled to 0°C followed by addition of pyridine (4.60 mL, 56.4 mmol), then DIC (6.59 mL, 42.3 mmol). The reaction was removed from ice bath immediately after addition of DIC was complete. The reaction was complete after 7.5 hours. The reaction mixture was diluted with 100 mL DCM. The organic layer was washed with 0.5 M HCl aq. (2x20 mL). The resulting organic layer was washed with water, brine, dried over MgSO₄, filtered and concentrated. The resulting DCM filtrate was slowly added dropwise to 300 mL of stirred hexanes at room temperature. The resulting solids were collected via vacuum filtration with aide of hexanes and dried on on frit filter overnight to afford off-white solids (6.5 g, 13.25 mmol, 47.0% yield, 93% ee). ESI-MS (EI⁺, *m*/*z*): 491.33, ¹H NMR (DMSO-*d₆*) δ: 11.35 (s, 1H), 9.10 (s, 1H), 9.05 (s, 2H), 8.91 (s, 1H), 8.36 (s, 1H), 5.85 (d, *J* = 7.2 Hz, 1H), 4.30 (s, 1H), 3.59 (d, *J* = 27.6 Hz, 2H), 3.46 (s, 3H), 3.19 (s, 3H), 2.08 (s, 3H), 1.95 (s, 1H), 1.88 (d, *J* = 7.3 Hz, 3H), 1.72 (s, 1H), 1.24 (d, *J* = 6.3 Hz, 3H)

### Example 12: Synthesis of a Comparator Compound

Sodium (2-(1,3-dimethyl-2,6-dioxo-1,2,3,6-tetrahydro-7*H*-purin-7-yl)-*N*-(6-(2-(2,2-dimethylpyrrolidin-1-yl)pyrimidin-5-yl)pyrazin-2-yl)acetamido)methyl hydrogen phosphate

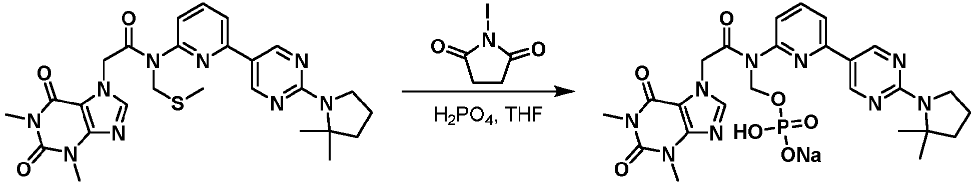

The title product was prepared from 2-(1,3-dimethyl-2,6-dioxo-1,2,3,6-tetrahydro-7*H-*purin-7-yl)-*N*-(6-(2-(2,2-dimethylpyrrolidin-1-yl)pyrimidin-5-yl)pyrazin-2-yl)-N-((methylthio)methyl)acetamide (Intermediate 9, 10.0 g, 10.1 mmol) using a procedure analogous to the one described for the synthesis of Example 9 Yield: 3.38 g (5.69 mmol, 31%); ¹H NMR (300 MHz, Deuterium Oxide) δ 8.79 (s, 1H), 8.75 (s, 2H), 8.56 (s, 1H), 7.83 (s, 1H), 5.51 (d, *J* = 7.0 Hz, 2H), 5.37 (br s, 2H), 3.54 (m, 2H), 3.32 (s, 3H), 3.14 (s, 2H), 1.88 (m, 4H), 1.41 (s, 6H); *m*/*z* [M+H]⁺ = 601.26.

## Claims

1. A compound of Formula (Ia), or a pharmaceutically acceptable salt thereof: 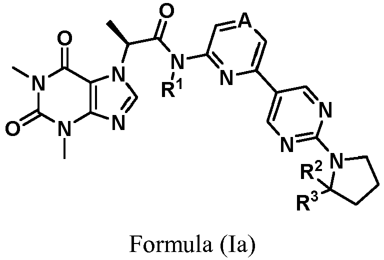 wherein,
R¹ is hydrogen;
R² is hydrogen or CH₃;
R³ is CH₃ or CF₃; and
A is N or CH.

2. The compound of claim 1, or a pharmaceutically acceptable salt thereof, wherein R² is hydrogen and R³ is CF₃.

3. The compound of claim 2, or a pharmaceutically acceptable salt thereof, wherein A is CH.

4. The compound of claim 1, or a pharmaceutically acceptable salt thereof, wherein R² is CH₃ and R³ is CH₃.

5. The compound of claim 2 or 4, or a pharmaceutically acceptable salt thereof, wherein A is N.

6. The compound of claim 1, or a pharmaceutically acceptable salt thereof, wherein R² is hydrogen and R³ is CH₃.

7. The compound of claim 1, wherein the compound is selected from a group consisting of:
(*S*)-(2-(1,3-dimethyl-2,6-dioxo-1,2,3,6-tetrahydro-7*H*-purin-7-yl)-*N*-(6-(2-(2,2-dimethylpyrrolidin-1-yl)pyrimidin-5-yl)pyrazin-2-yl)propanamide;
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyridin-2-yl)propanamide;
(*S*)-2-(1,3-dimethyl-2,6-dioxo-1,2,3,6-tetrahydro-7*H*-purin-7-yl)-*N*-(6-(2-((S)-2-methylpyrrolidin-1 -yl)pyrimidin-5 -yl)pyrazin-2-yl)propanamide; and
(*S*)-2-(1,3-dimethyl-2,6-dioxo-1,2,3,6-tetrahydro-7*H*-purin-7-yl)-*N*-(6-(2-((S)-2-(trifluoromethyl)pyrrolidin-1-yl)pyrimidin-5-yl)pyrazin-2-yl)propanamide;
or a pharmaceutically acceptable salt thereof.

8. The compound of claim 7, wherein the compound is selected from a group consisting of:
(*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyridin-2-yl)propanamide;
(*S*)-2-(1,3-dimethyl-2,6-dioxo-1,2,3,6-tetrahydro-7*H*-purin-7-yl)-*N*-(6-(2-((*S*)-2-methylpyrrolidin-1-yl)pyrimidin-5-yl)pyrazin-2-yl)propanamide; and
(*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamide;
or a pharmaceutically acceptable salt thereof.

9. The compound of claim 8, wherein the compound is (*S*)-2-(1,3-dimethyl-2,6-dioxo-1,2,3,6-tetrahydro-7*H*-purin-7-yl)-*N*-(6-(2-((*S*)-2-(trifluoromethyl)pyrrolidin-1-yl)pyrimidin-5-yl)pyrazin-2-yl)propanamide, or a pharmaceutically acceptable salt thereof.

10. A pharmaceutical composition comprising the compound of any one of claims 1 - 9.

11. A compound of any one of claims 1 - 9 or the pharmaceutical composition of claim 10 for use in a method for treating pain or providing analgesia.

12. The compound or composition for use of claim 11, wherein:
(a) the compound or composition is intravenously or orally administered and the compound is a compound of claim 1, optionally wherein the pain is post-surgical pain or chronic pain; or
(b) the pain is inflammatory pain.

13. A pharmaceutical composition comprising a therapeutically effective amount of the compound of any one of claims 1 - 9 for use in a method for treating or ameliorating pulmonary disease in an animal or human.

14. The composition for use of claim 13, wherein
(a) the pulmonary disease is asthma, optionally wherein asthma is allergic asthma; or
(b) the pulmonary disease is chronic obstructive pulmonary disease (COPD).

## Patentansprüche

1. Eine Verbindung der Formel (la) oder ein pharmazeutisch annehmbares Salz davon: 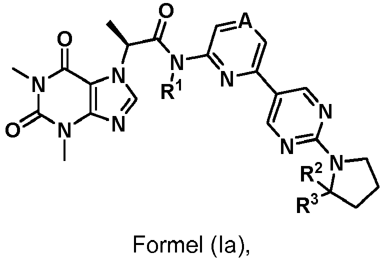 wobei
R¹ Wasserstoff ist,
R² Wasserstoff oder CH₃ ist,
R³ CH₃ oder CF₃ ist, und
A N oder CH ist.

2. Die Verbindung nach Anspruch 1 oder ein pharmazeutisch annehmbares Salz davon, wobei R² Wasserstoff ist und R³ CF₃ ist.

3. Die Verbindung nach Anspruch 2 oder ein pharmazeutisch annehmbares Salz davon, wobei A CH ist.

4. Die Verbindung nach Anspruch 1 oder ein pharmazeutisch annehmbares Salz davon, wobei R² CH₃ ist und R³ CH₃ ist.

5. Die Verbindung nach Anspruch 2 oder 4 oder ein pharmazeutisch annehmbares Salz davon, wobei A N ist.

6. Die Verbindung nach Anspruch 1 oder ein pharmazeutisch annehmbares Salz davon, wobei R² Wasserstoff ist und R³ CH₃ ist.

7. Die Verbindung nach Anspruch 1, wobei die Verbindung ausgewählt ist aus einer Gruppe bestehend aus:
(*S*)-(2-(1,3-Dimethyl-2,6-dioxo-1,2,3,6-tetrahydro-1*H*-purin-7-yl)-*N*-(6-(2-(2,2-dimethylpyrrolidin-1-yl)pyrimidin-5-yl)pyrazin-2-yl)propanamid,
(*S*)-2-(1,3-Dimethyl-2,6-dioxo-1,2,3,6-tetrazhydro-7*H*-purin-7-yl)-*N*-(6-(2-((S)-2-(trifluormethyl)pyrrolidin-1-yl)pyrimidin-5-yl)pyridin-2-yl)propanamid,
(*S*)-2-(1,3-Dimethyl-2,6-dioxo-1,2,3,6-tetrahydro-7H-purin-7-yl)-*N*-(6-(2-((S)-2-methylpyrrolidin-1-yl)pyrimidin-5-yl)pyrazin-2-yl)propanamid und
(*S*)-2-(1,3-Dimethyl-2,6-dioxo-1,2,3,6-tetrahydro-7*H*-purin-7-yl)-*N*-(6-(2-((S)-2-(trifluormethyl)pyrrolidin-1-yl)pyrimidin-5-yl)pyrazin-2-yl)propanamid,
oder ein pharmazeutisch annehmbares Salz davon.

8. Die Verbindung nach Anspruch 7, wobei die Verbindung ausgewählt ist aus einer Gruppe bestehend aus:
(*S*)-2-(1,3-Dimethyl-2,6-dioxo-1,2,3,6-tetrahydro-7*H*-purin-7-yl)-*N*-(6-(2-((*S*)-2-(trifluormethyl)pyrrolidin-1-yl)pyrimidin-5-yl)pyridin-2-yl)propanamid,
(*S*)-2-(1,3-Dimethyl-2,6-dioxo-1,2,3,6-tetrahydro-7*H*-purin-7-yl)-*N*-(6-(2-((*S*)-2-methylpyrrolidin-1-yl)pyrimidin-5-yl)pyrazin-2-yl)propanamid und
(*S*)-2-(1,3-Dimethyl-2,6-dioxo-1,2,3,6-tetrahydro-7*H*-purin-7-yl)-*N*-(6-(2-((*S*)-2-(trifluormethyl)pyrrolidin-1-yl)pyrimidin-5-yl)pyrazin-2-yl)propanamid,
oder ein pharmazeutisch annehmbares Salz davon.

9. Die Verbindung nach Anspruch 8, wobei die Verbindung (*S*)-2-(1,3-Dimethyl-2,6-dioxo-1,2,3,6-tetrahydro-7*H*-purin-7-yl)-*N*-(6-(2-((*S*)-2-(trifluormethyl)pyrrolidin-1-yl)pyrimidin-5-yl)pyrazin-2-yl)propanamid ist, oder ein pharmazeutisch annehmbares Salz davon.

10. Eine pharmazeutische Zusammensetzung, umfassend die Verbindung nach einem der Ansprüche 1 - 9.

11. Eine Verbindung nach einem der Ansprüche 1 - 9 oder die pharmazeutische Zusammensetzung nach Anspruch 10 zur Verwendung bei einem Verfahren zur Behandlung von Schmerz oder zur Schmerzlinderung.

12. Die Verbindung oder Zusammensetzung zur Verwendung nach Anspruch 11, wobei:
(a) die Verbindung oder Zusammensetzung intravenös oder oral verabreicht wird und die Verbindung eine Verbindung nach Anspruch 1 ist, gegebenenfalls wobei der Schmerz postoperativer Schmerz oder chronischer Schmerz ist, oder
(b) der Schmerz Entzündungsschmerz ist.

13. Eine pharmazeutische Zusammensetzung, die eine therapeutisch wirksame Menge der Verbindung nach einem der Ansprüche 1 - 9 umfasst, zur Verwendung bei einem Verfahren zur Behandlung oder Verbesserung einer Lungenerkrankung bei einem Tier oder Menschen.

14. Die Zusammensetzung zur Verwendung nach Anspruch 13, wobei
(a) die Lungenerkrankung Asthma ist, gegebenenfalls wobei das Asthma allergisches Asthma ist, oder
(b) die Lungenerkrankung chronisch obstruktive Lungenerkrankung (COPD) ist.

## Revendications

1. Composé de la Formule (Ia), ou un sel pharmaceutiquement acceptable de celui-ci: 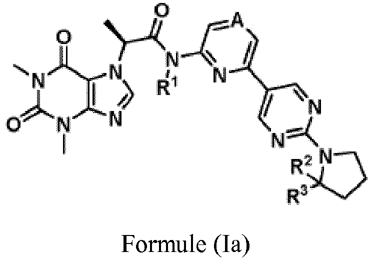 dans lequel,
R¹ est un hydrogène;
R² est un hydrogène ou CH₃;
R³ est CH₃ ou CF₃; et
A est N ou CH.

2. Composé selon la revendication 1, ou un sel pharmaceutiquement acceptable de celui-ci, dans lequel R² est un hydrogène et R³ est CF₃.

3. Composé selon la revendication 2, ou un sel pharmaceutiquement acceptable de celui-ci, dans lequel A est CH.

4. Composé selon la revendication 1, ou un sel pharmaceutiquement acceptable de celui-ci, dans lequel R² est CH₃ et R³ est CH₃.

5. Composé selon la revendication 2 ou 4, ou un sel pharmaceutiquement acceptable de celui-ci, dans lequel A est N.

6. Composé selon la revendication 1, ou un sel pharmaceutiquement acceptable de celui-ci, dans lequel R² est un hydrogène et R³ est CH₃.

7. Composé selon la revendication 1, où le composé est choisi dans le groupe constitué de:
(S)-(2-(1,3-diméthyl-2,6-dioxo-1,2,3,6-tétrahydro-7H-purin-7-yl)-N-(6-(2-(2,2-diméthylpyrrolidin-1-yl)pyrimidin-5-yl)pyrazin-2-yl)propanamide,
(S)-2-(1,3-diméthyl-2,6-dioxo-1,2,3,6-tétrahydro-7H-purin-7-yl)-N-(6-(2-((S)-2-(trifluorométhyl)pyrrolidin-1 -yl)pyrimidin-5 -yl)pyridin-2-yl)propanamide;
(S)-2-(1,3-diméthyl-2,6-dioxo-1,2,3,6-tétrahydro-7H-purin-7-yl)-N-(6-(2-((S)-2-méthylpyrrolidin-1-yl)pyrimidin-5-yl)pyrazin-2-yl)propanamide; et
(S)-2-(1,3-diméthyl-2,6-dioxo-1,2,3,6-tétrahydro-7H-purin-7-yl)-N-(6-(2-((S)-2-(trifluorométhyl)pyrrolidin-1-yl)pyrimidin-5-yl)pyrazin-2-yl)propanamide,
ou un sel pharmaceutiquement acceptable de ceux-ci.

8. Composé selon la revendication 7, où le composé est choisi dans le groupe constitué de:
(S)-2-(1,3-diméthyl-2,6-dioxo-1,2,3,6-tétrahydro-7H-purin-7-yl)-N-(6-(2-((S)-2-(trifluorométhyl)pyrrolidin-1 -yl)pyrimidin-5 -yl)pyridin-2-yl)propanamide;
(S)-2-(1,3-diméthyl-2,6-dioxo-1,2,3,6-tétrahydro-7H-purin-7-yl)-N-(6-(2-((S)-2-méthylpyrrolidin-1-yl)pyrimidin-5-yl)pyrazin-2-yl)propanamide; et
(S)-2-(1,3-diméthyl-2,6-dioxo-1,2,3,6-tétrahydro-7H-purin-7-yl)-N-(6-(2-((S)-2-(trifluorométhyl)pyrrolidin-1-yl)pyrimidin-5-yl)pyrazin-2-yl)propanamide,
ou un sel pharmaceutiquement acceptable de ceux-ci.

9. Composé selon la revendication 8, où le composé est le (S)-2-(1,3-diméthyl-2,6-dioxo-1,2,3,6-tétrahydro-7H-purin-7-yl)-N-(6-(2-((S)-2-(trifluorométhyl)pyrrolidin-1-yl)pyrimidin-5-yl)pyrazin-2-yl)propanamide, ou un sel pharmaceutiquement acceptable de celui-ci.

10. Composition pharmaceutique comprenant le composé selon l'une quelconque des revendications 1-9.

11. Composé selon l'une quelconque des revendications 1-9 ou composition pharmaceutique selon la revendication 10 pour une utilisation dans une méthode pour traiter une douleur ou donner une analgésie.

12. Composé ou composition pour une utilisation selon la revendication 11, où:
(a) le composé ou la composition est administré par voie intraveineuse ou orale et le composé est un composé selon la revendication 1, optionnellement où la douleur est une douleur post-chirurgicale ou une douleur chronique; ou
(b) la douleur est une douleur inflammatoire.

13. Composition pharmaceutique comprenant une quantité thérapeutiquement efficace du composé selon l'une quelconque des revendications 1-9 pour une utilisation dans une méthode pour traiter ou améliorer une maladie pulmonaire chez un animal ou un humain.

14. Composition pour une utilisation selon la revendication 13, où:
(a) la maladie pulmonaire est un asthme, optionnellement où l'asthme est un asthme allergique; ou
(b) la maladie pulmonaire est une maladie pulmonaire obstructive chronique (COPD).
